(19) Europäisches Patentamt / European Patent Office / Office européen des brevets

(11) **EP 2 136 771 B1**

(12) **EUROPEAN PATENT SPECIFICATION**

(45) Date of publication and mention of the grant of the patent:
**03.06.2015 Bulletin 2015/23**

(51) Int Cl.:
***A61K 8/23*** *(2006.01)*      ***A61K 8/60*** *(2006.01)*
***A61Q 19/08*** *(2006.01)*

(21) Application number: **08718012.1**

(22) Date of filing: **19.03.2008**

(86) International application number:
**PCT/EP2008/053286**

(87) International publication number:
**WO 2008/113819 (25.09.2008 Gazette 2008/39)**

(54) **COMPOSITIONS CONTAINING N-ACETYLGLUCOSAMINE FOR USE IN DERMO-COSMETOLOGY AND AESTHETIC MEDICINE**

N-ACETYLGLUCOSAMIN ENTHALTENDE ZUSAMMENSETZUNGEN ZUR VERWENDUNG IN DER DERMOKOSMETOLOGIE UND ÄSTHETISCHEN MEDIZIN

COMPOSITIONS CONTENANT DE LA N-ACÉTYLGLUCOSAMINE POUR UNE UTILISATION EN DERMOCOSMÉTOLOGIE ET EN MÉDECINE ESTHÉTIQUE

(84) Designated Contracting States:
**AT BE BG CH CY CZ DE DK EE ES FI FR GB GR HR HU IE IS IT LI LT LU LV MC MT NL NO PL PT RO SE SI SK TR**

(30) Priority: **22.03.2007 IT TO20070210**

(43) Date of publication of application:
**30.12.2009 Bulletin 2009/53**

(73) Proprietor: **Rottapharm S.p.A.**
**20122 Milano (IT)**

(72) Inventors:
• **SENIN, Paolo**
**I-20052 Monza (IT)**
• **SANTORO, Antonino**
**I-20052 Monza (IT)**
• **ROVATI, Luigi Angelo**
**I-20052 Monza (IT)**

(74) Representative: **Rambelli, Paolo et al**
**Jacobacci & Partners S.p.A.**
**Corso Emilia 8**
**10152 Torino (IT)**

(56) References cited:
**EP-A- 1 075 836**      **EP-A- 1 384 482**
**US-A- 3 697 652**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

**Description**

**Introduction**

Skin structure

Differently to as it may seem, skin has a rather complex structure and may be schematically likened to the overlapping of three tissue layers, namely the epidermis, dermis and cutaneous tissue, each characterised by precise, well-differentiated functions.

The upper layer, known as epidermis, is somewhat resistant and appears thin under the microscope. It progressively wears out and is constantly renewed. It is translucent and allows light to only partially pass through, rather like ground glass. The epidermis doesn't contain any blood vessels, it receives oxygen and nutrients from the deeper layers of the cutaneous tissue, impedes excessive loss of moisture from the body and gives healthy skin an attractive look.

All the cells of the epidermis originate from a single layer known as the basal layer. The dominant cell type in the epidermis is the keratinocyte, which takes its name from its ability to synthesize keratin. Keratins are non-water soluble natural proteins with high resistance to temperature and pH; they are divided into hard and soft keratins: the hard keratins from the hair, skin and nails, while the soft keratins are the main components of the cornified cells of the outermost layers of the epidermis, and are also found, as connecting substances, in the extracellular space of other layers of the epidermis.

Besides the keratinocytes, the epidermis also contains melanocytes, which are localised in the basal layer and produce the skin pigment known as melanin, which, depending on the amount, determines the colour of skin and hair; furthermore, melanocytes increase the expression of melanin as a result of the effect of solar radiation as a defence reaction against potential damage caused by the impact of ultraviolet rays on skin tissue.

The already-mentioned basal layer, separating the dermis from the epidermis, consists precisely of melanocytic cells and cylindrical keratinocytes, in charge of cellular mitosis, guaranteeing the continuous epidermal regeneration, and the cellular division of which depends, in turn, on the role played by other substances such as the various growth factors, hormones and assorted vitamins.

Between the basal layer of the epidermis and the dermis is located the basal membrane (itself also devoid of blood vessels), also known as the dermo-hypodermic junction which, besides separating the two cutaneous layers, is also involved in anchoring basal cells to the dermis and mediating various nutritional and metabolic functions.

The second layer, *i.e.* the dermis, contains blood vessels, nerves, the hair roots, sweat glands and all the structures conferring strength and elasticity to the skin.

The dermis is mainly composed of horizontal collagen bundles running across it and immersed in gelatinous substance known as fundamental substance, which in turn forms part of the extracellular matrix. Collagen constitutes up to 75% of the weight of the dermis and is responsible for the tonicity and elasticity of the skin. The collagen bundles are held together by elastic fibres made of a protein called elastin, which represents less than 5% of the weight of the dermis and, despite the name, is not directly responsible for the natural elasticity of skin.

Both the collagen and the elastic fibres are produced by cells known as fibroblasts, which are found in the dermis. Fibroblasts not only produce and organise the extracellular matrix of the dermis, but also communicate between one another and with other cell types, performing very important functions in the physiology of the skin such as, for example, the release of growth factors/cytokines which, in turn, play a significant role in wound healing by modulating keratinocyte activity (Sorrell M. and Caplan AI 2004).

Hyaluronic acid (HA) is another fundamental component of elastoviscous extracellular matrix in which the collagen fibres, elastic fibres and other cellular structures are immersed. It has the capacity to attract water in quantities equal to hundreds of times its weight, and thus represents a natural hydrating substance, responsible for the tonicity of the skin and its reserves of moisture. Furthermore, HA facilitates the transport of essential nutrients from the blood to skin cells. HA is a natural, linear polysaccharide composed of a disaccharide structural unit constituted by D-glucuronic acid and N-acetyl-glucosamine (NA) monosaccharides, and is present in all living organisms. Unlike collagen, hyaluronic acid shows no tissue or species specificity and is neither allergenic or irritant.

Another class of substances present in extracellular matrix, and closely structurally and functionally associated with HA, is represented by the glucosaminoglycans (GAGs). These are constituted by long chains of disaccharidic units wherein the monomers are represented by glucosamine or galactosamine and uronic acids. GAGs carry negative charges, due to the presence of sulphonic groups and the aforementioned uronic acids (and this structure explains their powerful capacity to attract negative ions and enormous quantities of $H_2O$), and become attached to protein chains (core proteins) to form the proteoglycans. Proteoglycans are the major component of extracellular matrix and, together with HA, to which they are covalently bound (by means of link proteins), and collagen fibres, constitute the extracellular structure of connective tissue, and hence skin, conferring said tissue with the majority of its characterising mechanical/functional characteristics.

Finally, the innermost part of the skin is represented by the hypodermis or subcutaneous layer, consisting of blood vessels, nerves and adipocyte clusters. From the structural viewpoint, the separation from the overlying dermis is not well defined, while deeper, the hypodermis is bound to the underlying muscle and adipose tissue, which is deposited therein in varying amounts, and exerts a well defined isolating and modelling function.

Functional characteristics of skin and conditioning factors

Various studies have been conducted with the scope of determining the effect of hyaluronic acid and other substances on skin cell activity.

- Exogenous hyaluronic acid can influence skin fibroblast proliferation, and this effect varies depending on the cell density and the concentration of hyaluronic acid itself. In this regard, and as observed by Yoneda M. *et al.*(1988), the addition of HA to primary cultures of mouse skin fibroblasts, causes transient DNA synthesis with consequent positive backup constituted by an increase in skin fibroblasts themselves, and hence the expression of endogenous HA.
- The importance of exogenous HA is further confirmed by the fact that, according to Isnard N *et al.* (2001), in cultures of human fibroblasts and keratinocytes, the addition of HA (1 mg/ml) causes a clear increase in the expression of extracellular matrix metalloproteases, known to play an important role in tissue remodelling in a variety of physiological and pathological processes.

With advancing age, skin elasticity and tone reduces visibly with the consequent appearance of wrinkles of various depths and loss of the turgor characteristic of younger more aesthetically pleasing skin. This ageing is essentially associated with slowed turnover of both HA, with consequently reduced capacity to absorb/retain tissue moisture, and collagen, with deterioration of the other functional characteristics of skin, such as resistance to external stress and elasticity.

The search for substances to be used in dermo-cosmetology and aesthetic medicine for topical use or as a skin filler for the correction of facial skin wrinkles and for enhancement of soft facial tissue, has been subject to continual effort for a number of years. Various biomaterials are currently available, but all have shown burdensome limitations: they are either absorbed too quickly and are thus of no practical use, or they can give rise to allergic reactions (such as for example in the case of collagen), or even migrate away from the site of injection.

A safe and effective substance for the above-mentioned uses must be biocompatible, non-pyrogenic, must be neither allergenic nor toxic, must not cause inflammation, must be easy to use, stable and not migrate following injection, must last for as long as possible but at the same time, must be reabsorbable and must give the skin a natural look: due to its physico-chemical and mechanical/structural characteristics, hyaluronic acid has shown itself to be able to simultaneously satisfy all these functional requirements. It was developed as a skin filler for the first time in 1989 by E. Balazs, who observed the biocompatibility and absence of immunogenicity (Balazs E.A. and Leshchiner E.A. 1989). Exogenous hyaluronic acid is quickly reabsorbed by the dermis and metabolised in the liver with the formation of carbon dioxide and water. The HA reabsorption process is rapid and complete, and depends on receptor binding and intracellular degradation. The half-life in the skin is very short, *i.e.* less than 24 hours.

In order to obviate said drawback, hyaluronic acid for use as a prolonged effect filler may be chemically cross-linked. While keeping unaltered its biocompatibility, the cross-linking process alters the solubility and rheological properties of this polysaccharide, becoming more viscous and assuming the consistency of a gel. Hyaluronic acid gels used as skin fillers are "hydrogels", since they are reswollen by 95% of their weight in water and remain stable in tissue, being reabsorbed only after several months, thus making them advantageous for use in dermo-cosmetology and aesthetic medicine.

A further advantage is represented by the fact that, unlike other temporary fillers such as collagen, hyaluronic acid-based gels are eliminated from the tissue by isovolumic degradation and that, little by little, as the molecules of hyaluronic acid are degraded and eliminated, the residues can bind more water, with the advantage that the entire volume injected remains unchanged.

To complete the picture on the use of hyaluronic acid in dermo-cosmetology, it should be underlined that, in its non-cross-linked form, it may be advantageously used both as it is, and in association with other active ingredients, in solutions, gels, creams or other forms for topical application, with obvious beneficial effects on skin tone and turgor, promoting hydration and thus aesthetic appearance.

The results of various clinical trials published in the literature, on various hyaluronic acid gels are generally in agreement: indeed, both from the viewpoint of the physician and the patient, very satisfactory improvements are observed with skin defects, and the degree of correction has been evaluated as between 60 and 90%, 6-9 months after the first injection (Duranti F. *et al.* 1998; Olenius M. 1998; Carruthers J. *et al.,* 2005; S. di *Bosniak et al.,* 2004; Narins R. S. *et al.* 2003; Lindqvist C *et al.,* 2005; Carruthers J. & Carruthers A., 2003).

As already mentioned previously, among the fundamental components of the extracellular matrix of connective tissue and hence skin, we find the glucosaminoglycans (GAGs) and hyaluronic acid, and the characteristics, properties and functions of the latter have already been exhaustively described and specified.

In both cases, from the structural viewpoint, these are unbranched glucosidic polymers, wherein the repetitive unit is constituted by disaccharides, the monomers of which are represented by uronic acids such as glucuronic, galacturonic or iduronic acid and aminosugars such as N-acetylglucosamine or N-acetyl galactosamine, variously substituted.

From the above, it is simple to deduce that, besides HA, there are other substances that may be extremely important in preserving and/or improving skin functionality and aesthetic appearance. One of these is NAG.

There are studies available in the literature and tests, described below in the experimental section of the present patent application, showing how NAG exerts an indisputable positive action on skin characteristics and functionality, so as to justify and recommend its advantageous use in dermo-cosmetology and aesthetic medicine. Thus, for example:

- NAG stimulates the activity of the enzyme hyaluronate synthetase in the plasma membrane fractions of human skin fibroblasts (Mian 1986), thus supporting and synergising the use of exogenous HA in defending the skin against external stress and natural and/or pathogenic ageing processes with consequent loss of tone, turgor, luminosity and the appearance of wrinkles of varying depth.
- In an *"in vitro"* model reproducing the various skin layers, *i.e.* MatTek Human Skin EpiDemFT skin model(MakTek Corp., Ashland, MA USA), the addition of NAG caused a very high, significant and dose dependent increase of HA and pro-collagen 1 (Osborne R *et al.* 2006).
- To complete the existing scientific documentation and from the experimental section detailed below, it is evident that, in *"in vitro"* cultures of fibroblasts and keratinocytes of human origin, NAG exerts a clearly dose-dependent effect on the expression of HA (tested on fibroblasts alone), collagen, elastin and other proteins normally produced in the dermis, justifying and further supporting its use in dermo-cosmetology, in accordance with the methods described, exemplified and claimed in the present patent application.

US-A-3697652 discloses N-acetylglucosamine and sodium sulphate in pharmaceutical preparations for the treatment of degenerative afflictions of the joints.

EP-A-1384482 discloses skin care compositions comprising N-acetylglucosamine and at least one of retinoid or pro-vitamin A.

EP-A-1075836 discloses the use of N-acetylglucosamine as a skin care agent for oral administration.

Summary of the invention

From the description provided in the introduction, it is clearly obvious that the use of both exogenous HA and NAG is certainly effective and to be recommended in preparations for topical (dermo-cosmetology) and intradermal (aesthetic medicine) use.

The present invention is based on recognition of the fact that NAG activity is potentiated thanks to the association between NAG and an alkaline metal sulphate within a defined weight ratio range. Thus, a synergic composition, as defined in the following claims, constitutes the subject of the invention.

According to the invention, a molecular composition between NAG and an alkaline metal sulphate (hereinafter anhydrous sodium sulphate (ASS) will be used as ponderal reference) has been realised and studied, wherein the equivalent mass ratio between NAG and ASS may vary between 1:0.5 and 1:3, then practically using, for reasons that will be clarified in the experimental section, the most advantageous ratio, i.e. 1:1 (corresponding in ponderal terms to 75.7% NAG and 24.3% ASS). With regard to this subject, it should be highlighted that, in order to avoid confusion, hereinafter, the molecular combination corresponding to the experimentally most advantageous ratio between equivalent masses, i.e. 1:1 will be known as Condramina (CA) while all other combinations in the range 1:0,5 - 1:3 (obviously excluding 1:1), will be identified by the abbreviation COMBI. After a preliminary experimental stage, revealing the advantageous use of CA with respect to all the other COMBI preparations (in any case convenient with respect to the use of NAG at corresponding doses), the activities of CA in stimulating the expression of HA, collagen, elastin and other proteins, in "in vitro" cultures of fibroblasts and keratinocytes, have been tested and compared with those of NAG and sodium sulphate, taken individually and in doses/concentrations corresponding to those present in CA, used in the same tests.

- As expected, and as described in detail in the experimental section, NAG gave optimal results both in cultures of fibroblasts and keratinocytes while, in a similarly predictable manner, sodium sulphate was shown to be entirely inactive, hence, with results not significantly distinguishable from those of the control cultures.

- In an entirely unexpected and unpredictable manner, except in the case in the stimulation of elastin expression in fibroblast cultures, where there is no significant difference with NAG, CA invariably gave better results than those of NAG at the corresponding doses/concentrations. Such effects were consistently shown to be dose-dependent and statistically significant, leading to the conclusion, entirely unpredictable beforehand, of the undeniable existence of a synergistic action between NAG and ASS in exerting a useful stimulation of the production of substances useful to cutaneous trophism by those cells characteristic of cutaneous tissue itself, such as fibroblasts and keratinocytes.

- Moreover, as the concentrations/doses, used in the tests are absolutely compatible with potential practical applications and parallel tests on the potential CA cytotoxicity have given quite reassuring results, there is an obvious practical possibility of advantageously using CA, in place of NAG, both for oral use and for topical and intradermal use, preferably in association with HA, in dermo-cosmetology and aesthetic medicine as described in detail in the examples described below in the present patent application.

**Information on the active ingredients used in the compositions described and claimed in the present patent application**

Hyaluronic acid (HA) used as it is, or as a sodium salt:

- CAS No. (acid): 9004-61-9
- CAS No. (sodium salt): 9067-32-7
- Origin: biofermentation
- Molecular weight: comprised of between 0.5 and $3 \times 10^6$ Da
- Level of cross-linking (where used) : comprised of between 0.5 and 5%

The concentrations of HA described and claimed in the present patent application do not exceed 4% and are preferably comprised in the range 1-3%.

NAG-ASS combination (COMBI):

COMBI is composed of NAG and ASS in equivalent weight ratios varying between 1:0.5 and 1:3 (excluding the ratio of 1:1 already identified as Condramina (CA)), corresponding to ponderal ratios oscillating between 86.17% and 50.93% for NAG and between 13.83% and 49.07% for ASS:

Condramina (CAS):

CA is composed of NAG and ASS in an equivalent weight ratio equal to 1:1, corresponding in ponderal terms to 75.7% CA and 24.3% ASS

Both COMBI and CA are constituted by:

a. NAG:

- Name: N-acetyl-2-amino-2 deoxyglucose
- CAS No.: 7512-17-6
- Molecular weight: 221.19
- Empirical formula: $C_8H_{15}NO_6$

b. ASS:

- CAS No.: 7757-82-6
- Molecular weight: 142,05
- Empirical formula: $Na_2O_4S$

The concentrations/doses of CA used within the scope of the invention are preferably comprised of between 0.05% and 2.5% by weight, and more preferably between 0.1 and 0.5% with regard to the topical and intradermal forms while, if used for oral administration, CA may be taken in daily doses, expressed in terms of glucosamine base, comprised of between 100 and 1000 and preferably 250-750 mg, in one or more administrations, depending on the dose and pharmaceutical form used.

**Experimental section**

The synergistic effect achieved by the composition according to the invention has been verified through a series of "in vitro" tests, evaluating and comparing the experimental results in relation to Condramina (CA) with those of its components, *i.e.* N-acetylglucosamine (NAG) and anhydrous sodium sulphate (ASS) considered individually, at concentrations consistent with the ratio between their equivalent weights in CA, shown to be the most convenient *i.e.* 1:1. Said ratio has been selected on the basis of the results of a preliminary test where the evaluation has been focussed on the experimental effect of the variation of the reciprocal ratio of NAG and ASS in COMBI.

**Methods**

The tests selected have been:

**1. In vitro evaluation of the stimulation of the synthesis of hyaluronic acid (HA) by CA, NAG and ASS in human fibroblast cultures (see also: Sayo *et al.* 2004)**

The test has been conducted in two stages, i.e.:

- stage A: in which the stimulation of HA expression by COMBI has been evaluated while maintaining the concentration constant and varying the reciprocal quantities of its components. The concentration used has been selected so as to enable comparison with the results derived from stage B;
- stage B: in which the effect of CA on HA expression has been evaluated by varying its concentration and in comparison with doses consistent with its components.

Test rationale

- Since the HA content of skin decreases with age, giving rise to skin ageing, with the consequent appearance of wrinkles and loss of elasticity, the scope of the test has been the evaluation and comparison of the potential stimulation, exerted by the test substances, on HA expression in "in vitro" cultures of skin derived cells such as fibroblasts. The test in question, even though conducted "in vitro", can be considered to be predictive of the effects of use of the same substances "in vivo".

Cellular model

Human fibroblasts, Detroit 551 ATCC CCL III from LGC Promochem (Milan, Italy) have been used.

Test substances and concentrations

The substances tested for their effect on the expression of HA in human fibroblast cultures have been:

Stage A:

- COMBI: at a constant concentration of 1.98 mg/ml with NAG to ASS ratios, in terms of equivalent weight equal to 1:0.4 ; 1:0.5 ; 1:3 and 1:3.5
- CA: at a concentration of 1.98 mg/ml with an equivalent weight ratio, between NAG and ASS, equal to 1:1

Stage B:

- CA: wherein the ratio between the components, in terms of equivalent weight, has been kept constant and equal to 1:1 and the concentrations tested are 0-66; 1.32; 1.98; 2.64 and 3.3 mg/ml
- NAG at concentrations of 0.5, 1.0, 1.5, 2.0 and 2.5, corresponding to the NAG content in CA at the concentrations and in the equivalent weight ratio (1:1) used in the same test

- ASS at concentrations of 0.16, 0.32, 0.48, 0.64 and 0.80 mg/ml, corresponding to the ASS content in CA at the concentrations and in the equivalent weight ratio (1:1) used in the same test

Preparation of cell cultures

Human fibroblasts have been grown in suitable growth medium (Eagle's minimal essential medium) supplemented with 10% foetal calf serum (FCS), 1 mM sodium pyruvate, 2 mM glutamine, non-essential aminoacids and 50 $\mu$g/ml glutamycin. Confluent cells have been plated in 24 well plates, $5\times10^4$ cells/well, using the same medium. At confluence, the cells have been pre-exposed to medium containing 5% serum, prior to the addition of the test substances at the desired concentrations. After 48 hours, the supernatant has been removed and used for the determination of the parameters of interest. The test has been conducted in duplicate and untreated cell cultures have been used as controls.

HA assay

HA has been assayed (dosed) by means of an immuno-enzymatic test using a commercially available kit (EIA, Corgenix). *De novo* HA synthesis has been evaluated in the culture medium following treatment with the test substances, according to the protocol provided with the test, and against the calibration curve obtained using the HA standard contained in the kit.

Evaluation of the results

Stage A:

- evaluation of effects of COMBI and CA on fibroblast cultures, at varying reciprocal ratios of their components within a constant maintained concentration, has been expressed as the absolute and percent increase or decrease of the HA expression with respect to the corresponding control culture after 48 hours of incubation, and by comparison with the effects exerted by NAG at the concentrations corresponding exactly or approximately to those present in CA and in COMBI, at varying reciprocal ratios between its NAG and ASS components.

Stage B:

- evaluation of the effects of the substances tested, at the various concentrations, on fibroblast cultures has been expressed as the absolute and percent increase or decrease of the HA expression, with respect to the corresponding control culture, after 48 hours of incubation.

The percentage difference increase or decrease of the effects exerted by CA and NAG, at corresponding concentrations, have then been calculated, with evaluation of their potential significance by means of the Student's "t" test.

**2. In vitro evaluation of the stimulation of the synthesis of collagen and elastin by CA, NAG and ASS in human fibroblast cultures (see also: *Booth et al.* 1980; Fenwick *et al.* 2001)**

Test rationale

The scope of the test has been the evaluation and the comparison of the potential stimulation exerted by the test substances on the expression of collagen and elastin in "in vitro" cultures of skin-derived cells, such as fibroblasts. The test in question, even though conducted "in vitro", can be considered to be predictive of the effects of use of the same substances "in vivo".

Cellular model

Human fibroblasts, Detroit 551 ATCC CCL III from LGC Promochem (Milan, Italy) have been used.

Test substances and concentrations

The substances and concentrations tested have been the same as those already described for stage B of the test on the stimulation of HA expression, *i.e.*:

- CA: 0.66, 1.32, 1.98, 2.64 and 3.3 mg/ml

- NAG: 0.5, 1.0, 1.5, 2.0 and 2.5 mg/ml

- ASS: 0.16, 0.32, 0.48, 0.64 and 0.80 mg/ml

Preparation of cell cultures

Cells have been plated out in 96 well plates, 2500 cells/well for 24 hours in cell growth medium (Dulbecco's Minimum Essential Medium, DMEM) + 10% foetal calf serum (FCS). Fresh culture medium, enriched with just 5% FCS and containing the substances to be tested, so as to reach the final concentrations desired, has then been added. The samples have been dissolved directly in the culture medium. Each sample has been tested in duplicate and the experiments have been repeated twice. The parameters of interest have been determined after 24 hours on separate plates. Untreated cell cultures have been used as controls.

Collagen assay

A commercially available kit (Sircol™, biodye science) which uses the capacity of the stain Sirius Red to interact with the basic side chains of the aminoacids present in collagen itself, has been used for collagen assays. *De novo* collagen synthesis has been evaluated in the culture medium following treatment with the test substances, following the protocol provided with the kit, and by using the calibration curve obtained employing the collagen standard contained in turn in the kit.

Elastin assay

Elastin has been assayed using a commercially available test (Fastin™, biodye science). The assay is based on the interaction of 5,10,15,20-tetraphenyl-21,23-porphyrin with elastin molecules.

*De novo* elastin synthesis has been evaluated in the culture medium following treatment with the test substances, following the protocol provided with the test, and by using the calibration curve obtained employing the elastin standard contained in the kit itself.

Evaluation of the results

Evaluation of the effects of the substances tested, at the various concentrations, on fibroblast cultures, has been expressed as the absolute and percent increase or decrease of collagen and elastin expression, with respect to the corresponding control culture, after 24 hours of incubation.

The percentage difference increase or decrease of the effects exerted by CA and NAG, at corresponding concentrations, have then been calculated, with consequent evaluation of their potential statistical significance by means of the test Student "t" test.

**3. "In vitro" evaluation of the stimulation of protein synthesis by CA and NAG in human fibroblast and keratinocyte cultures: (see also: *Lowry et al.* 1951; Creighton *et al.*1984; Sengupta *et al.* 1993)**

Preliminary information

Fibroblasts and keratinocytes, present in the dermis and epidermis, besides being involved in the synthesis of collagen, elastin and HA, are also involved in the expression of various protein species including, for example, keratin by keratinocytes and the so-called core e link-proteins, onto which are attached various types of GAGs to form proteoglycans, which are in turn, fundamental components of extracellular matrix.

The level of viability and the efficiency of such cells, in the presence or absence of agents whose beneficial action towards skin it is desired to verify, may be evaluated and deduced from their protein synthetic capacity.

Cellular models

The following have been used:

- Human fibroblasts, Detroit 551 ATCC CCL III from LGC Promochem (Milan, Italy);
- Human keratinocyte primary culture: HEKA, Cascade Biologics, cat. 005-5C.

Test substances and concentrations

The substances and concentrations tested are those reported below (on both fibroblasts and keratinocytes):

- CA: 0.13, 0.66, 1.32, and 2.64 mg/ml

- NAG: 0.1, 0.5, 1.0, and 2.0 mg/ml

Test rationale

Based on the previously mentioned information relating to indicators of efficiency of cells present in the skin, the scope of the test has been the evaluation and comparison of the potential stimulation, exercised by the test substances, on protein expression in "in vitro" fibroblast and keratinocyte cultures.
The test in question, even though conducted "in vitro", can be considered to be predictive of the effects of use of the same substances "in vivo".

Preparation of cell cultures

The method is identical to that already described for collagen and elastin with the difference that, following the addition of the test substances into the culture medium, the medium has been substituted daily for three days. Each sample has been tested in duplicate and the experiments have been repeated twice. The parameter of interest, *i.e.* protein synthesis, has been evaluated at 24, 48 and 72 hours on separate plates. Untreated cell cultures have been used as controls.

Protein assay

Protein has been assayed using the Lowry method *[Lowry et al.* 1951] consisting of a Biuret reduction reaction envisaging the use of Folin-Ciocalteau reagent as chromophore, with a yellow to blue colour change and spectrophotometric measurement at 750 nm *[Creighton et al.* 1984; *Sengupta et al.* 1993].
The protein content of the cultures in question has been obtained from comparison of their optical densities with a titration curve made using albumin as a protein standard.

Evaluation of the results

Evaluation of the effect of the substances tested, at the various concentrations and times, on cultures of both fibroblasts and keratinocytes has been expressed in both absolute terms (expressed protein production in mg/ml) and as percentage increase or decrease with respect to control cultures.
The percentage difference increase or decrease of the effects exerted by CA and NAG, over various days and at the corresponding concentrations, have then been calculated, with evaluation of their potential statistical significance by means of the test Student "t" test.

**4. "In vitro" evaluation of the cytotoxic potential of substances for topical cosmetic and intradermal use: (see also: Mossman, 1993)**

Test rationale

The particular characteristics of substances to be used for topical or intradermal use in cosmetics must be absolutely free from any cytotoxic effects within the dose/concentration range for their intended practical application.
There are appropriate "in vitro" tests, conducted on cells derived from skin tissue, such as fibroblasts and keratinocytes, for predicting the potential cytotoxicity of test substances "in vivo".
For this purpose the cell viability test using MTT (3-[4,5-dimethylthiazol-2-yl]-2,5-diphenyl tetrazolium bromide) [Mossman 1993] a colourimetric reagent, by means of which it is possible to distinguish between living, damaged or dead cells, being thus predictive of the safety of use of test substances, even "in vivo", has been selected.

Cellular models

The following have been used:

- human fibroblasts, Detroit 551 ATCC CCL III from LGC Promochem (Milan, Italy);

- primary culture of human keratinocytes (HEKA), Cascade Biologics, cat. - 005-5c.

Test substances and concentrations

Since, in the composition forming the subject of the present invention, NAG and ASS are never claimed for individual use, but always in association in the composition of CA, the only substance tested for its potential cytotoxic effect has been CA, at such concentrations as to be predictive and referable to those used in "in vitro" activity testing on the previously described fibroblast and keratinocyte cultures.

In the study in question, CA has been previously dissolved and brought into contact with the selected cell cultures in such a manner as to directly reach the desired concentrations, i.e.:

0.026, 0.053, 0.106, 0.211, 0.409, 0.818, 1.65 and 3.30 mg/ml.

Analogous samples of untreated human fibroblasts and/or keratinocytes have been used as negative controls.

Analogous samples of human fibroblasts or keratinocytes have been treated with a surfactant of known activity (SDS), dissolved in culture medium at concentrations comprised of between 0.05 and $1.6 \times 10^{-3}$ mg/ml, and used as a positive control.

Exposure has been for a period of 24 hours, on completion of which, the cytotoxicity test has been conducted.

Description of the cytotoxicity test using MTT

As already mentioned, the key reagent is MTT, a yellow-coloured substance in aqueous solution which, when acted-on by the mitochondrial dehydrogenases present in viable cells, is transformed into purple crystals, insoluble in water, but soluble in acidified isopropanol. The absorbance of the resulting purple solution at 540 nm is used as an indicator of the level of cytotoxicity of the substances under test.

In operational terms, the key reagent is prepared by adding 15 mg of MTT to 30 ml of culture medium. Aside, after 24 hours in contact with the test substance and/or SDS, the fibroblasts or keratinocytes are washed with 400 $\mu$l of wash solution (Dulbecco's Phosphate Buffered Saline, DPBS) and, following removal of the solution, 200 $\mu$l of MTT medium added, and the cell samples incubated for 4 hours at 37°C. On completion of the incubation period, the MTT medium is removed and 400 $\mu$l of MTT solubilising solution added (10% Triton X-100 + 0.1 N HCl in anhydrous isopropanol). The plates are shaken for 20-30 minutes until a homogenous solution is formed, the absorbance of which is then read at 540 nm with a background reading at 670 nm.

Results are expressed as:

$$\% \text{ inhibition and cell viability} = \left[1 - \frac{OD \text{ cellule trattate}}{OD \text{ cellule non trattate}}\right] x100$$

Evaluation of the results

The cytotoxicity data, obtained using the MTT test, is plotted on a graph against the concentration of the product under test, thus giving a dose-response curve, allowing the determination of:

- the theoretical regression curve;

- the theoretical $IC_{50}$ value, or the concentration resulting in a 50% reduction in cell viability with respect to that of untreated cells.

The irritant potential of a compound is related to the $IC_{50}$ value according to the following evaluation criterion:

- $IC_{50} < 0.5$: strong cytotoxic/irritant effect

- $IC_{50}$ between 0.5 and 1.5: moderate cytotoxic/irritant effect

- $IC_{50} > 1.5$: absence of any cytotoxic and/or irritant effect

**Results**

**1. Stimulation of HA synthesis by CA, NAG and ASS in human fibroblast cultures**

Stage A:

From the results reported in Table 3, Stage A, it is obvious that there is clear synergism between NAG and ASS depending on the ratios, in terms of equivalent weight, varying between 1:0.5 and 1:3 with the most favourable verifiable ratio being 1:1. For combinations outwith said favourable range, no synergism is detectable, since the effect of the combination between NAG and ASS under such conditions is even less than that of NAG alone and at concentrations corresponding to that present in the combination itself. Indeed, it may be observed how for a NAG/ASS ratio equal to 1:0.4 (the most favourable to NAG), the increase in HA expression is approx. 65%, markedly inferior than that for NAG at the same concentration, which is certainly greater than 75%. Analogously, for a NAG/ASS ratio equal to 1:3.5 (the most favourable to ASS), the increase is approx. 50% compared to that of NAG alone, which approaches 60% at the same concentration.

Stage B:

From the results reported in tables 1 to 3, Stage B, it is possible to draw the following conclusions:

- ASS alone, in increasing doses corresponding to those present in CA, tested in the same experiment, exerts no stimulation nor inhibition, and in effect, at all concentrations tested, the differences with respect to the HA expression of the baseline culture are irrelevant, both in absolute and in percentage values. In confirmation of the above, from examination of the experimental data, it is not possible to identify any correlation between ASS concentration and HA expression, since the coefficient of correlation associated with the corresponding regression, calculated using the least squares method, is not statistically significant (r=0.345 with DoF=4).

- Unlike the findings of Sayo T *et al.,* 2004 for NAG, both CA and NAG, at the tested concentrations absolutely consistent as expression of the corresponding dosage of NAG shows a clear stimulation of HA secretion in human skin-derived fibroblast cultures. In both cases, said effect is very evident, with a difference already appearing at 0.66 mg/ml for CA (corresponding to 0.5 mg/ml NAG) while it is delayed to 1 mg/ml in the case of NAG. It is also observed that, in both cases, the intensity of the effect is dose-dependent (r=0.973 for NAG and 0.939 for CA, both statistically significant for the degrees of freedom of the corresponding correlations) and that HA expression appears, at all concentrations, more marked in the case of stimulation by CA with an increase equal to approx. 99% at 1.98 mg/ml (corresponding to 1.5 mg/ ml NAG), an increase only achieved by NAG, as it is, at the highest dose tested, *i.e.* 2.5 mg/ml.

- With reference to table 4, to evaluate the difference in stimulation of HA expression by CA and NAG, the difference has been calculated between percentage increases, at the corresponding doses of both test substances, and it has been confirmed that, at all concentrations, CA exerts a greater percentage of stimulation than NAG. By then applying the Student's "t" test to the differences between the above-mentioned percentage increases, it emerges that the value observed for "t" is highly significant, leading to the highly unforeseeable and surprising conclusion that a clear synergistic effect exists between ASS and NAG since, as already witnessed, ASS alone has no effect on fibroblast H, while on the other hand, there is a clear stimulatory action if associated with NAG in the composition of CA, making the functional and formulative use in place of NAG, if used alone, clearly and unexpectedly advantageous.

**2. Stimulation of collagen and elastin synthesis by CA, NAG and ASS in human fibroblast cultures**

A. Collagen

From the results reported in tables 5 -7 it is possible to draw the following conclusions:

- Again in this case, ASS, considered individually within the concentration range consistent with that used for the other 2 test substances, *i.e.* CA and NAG, exerts no type of stimulation or inhibition on the expression of collagen in human skin derived fibroblast cultures. Again in this case, for ASS, it is not possible to identify any kind of concentration/effect relationship, since the correlation coefficient of the regression between its concentration and collagen expression (r=0,298) is not statistically significant.
- Both CA and NAG, at the concentrations tested and entirely consistent as expression of the same dose of NAG, exert a clear stimulation effect on the secretion of collagen in human skin derived fibroblast cultures. In both cases, the intensity of this effect is very marked and dose-dependent, so that for both, it is possible to calculate a concentration/effect regression characterised by a high and statistically significant correlation coefficient (r=0.927 for stimulation by NAG and 0.929 for stimulation by CA).
- From the data in table 11, reporting the percentage increases with respect the baseline control, for the expression of collagen stimulated by CA and NAG, again at comparable levels, it emerges that the percentage stimulation effect from CA is greater than that from NAG at all concentrations tested. By then applying the Student's "t" test to the differences between the above-mentioned percentage increases, it emerges that the value determined for "t" is highly significant, thus highlighting, again in this case, the synergic and previously unexpected effect that exists between ASS and NAG in CA, thus confirming the advantageous functional and formulative use of the latter in place of NAG alone.

B. Elastin

From the results reported in tables 8 -10 it is possible to draw the following conclusions:

- As already verified in the case of the tests of the expression of collagen and HA, ASS, at concentrations consistent with those of the other two test substances, *i.e.* CA and NAG, is incapable of exerting any effect, either stimulatory or inhibitory, on the expression of elastin by human skin-derived fibroblasts.

On the other hand, both CA and NAG, at the concentrations tested and consistent with one another, exert a very marked stimulatory effect that, in both cases, is already apparent at the lowest concentration tested *i.e.* 0.5 mg/ml for NAG and 0.66 mg/ml for CA (corresponding to 0.5 mg/ml NAG) increasing in a dose-dependent manner until exceeding 300% with respect to the baseline control, at the maximum concentration *i.e.* 2.5 mg/ml NAG and 2.64 mg/ml CA (corresponding to 2.5 mg/ml NAG).

- From the data presented in table 12, where the test results are expressed as percentage increase with respect to baseline control, and contrary to the picture that emerges for tests relating to the expression of HA and collagen, in this case, it is not possible to identify any difference between the stimulatory actions exerted by CA and NAG. This experimental evidence is confirmed by the fact that the Student "t" test applied to the differences between the percentage increases in relation to the two active ingredients under test, shows no statistical significance. This trend, which does not confirm that already seen in terms of the effects on the expression of HA and collagen, where the stimulation exerted by CA is always greater, is most likely to be attributed to the high intensity of the stimulation itself, which causes an increased elastine expression equal to approx. 200% already at the lowest concentration, *i.e.* corresponding to 0.5 mg/ml NAG.

**3. Stimulation of protein synthesis by CA and NAG in human fibroblast and keratinocyte culture**

A. Keratinocytes

From the results reported in table 13 it is possible to draw the following conclusions:

- For both NAG and CA, stimulation of protein expression is observed at the highest concentrations, *i.e.* 1 and 2 mg/ml, after just 24 hours, also with considerable percentage increases *i.e.* over 50% for NAG, at the highest dose and almost 70% at the concentration corresponding to that for CA.

- As a consequence, the culmination of stimulation action by both test substances is observed after 48 hours when, unlike the observations at 24 hours, already at the lowest concentrations *i.e.* 0.1 and 0.5 mg/ml NAG and 0.66 CA, the increased protein expression by keratinocytes assumes a positive value of around 20% for NAG and even reaches 30-40% for CA. However, a concluding picture is obtained where both test substances show a clear stimulation of protein expression on keratinocytes at all concentrations tested and in a dose-dependent manner, as demonstrated by the statistical significance of the coefficients of correlation associated with concentration/effect regression, equal to 0.726 for NAG and 0.909 for CA.

- After 72 hours, there is still residual stimulatory action at the two highest concentrations, but it appears obvious that this effect is only the tail of the peak reached at 48 hours, and as such is not significant.

- With regard to the comparison of activities between CA and NAG, the differences between the increases in percentage stimulation exerted by the two test substances have been evaluated using the Student's "t" test, as already done for the experimental results of the other previously examined tests (see stimulation of expression of HA, collagen and elastin).

Particularly, by examining and comparing the actions exerted by the two substances at 48 hours, *i.e.* at the peak of their stimulatory effect (said comparison at 24 and 72 hours has little significance since it is either temporally premature or late) it is clear that CA exerts a markedly superior stimulatory action on protein expression in keratinocytes than that of NAG at corresponding doses. This experimental results is further confirmed by the Student "t" value, applied to the differences in percentage stimulation of the two test substances, which is highly significant.

Hence, once more, the unexpected and unforeseen synergic effect between ASS and NAG is proven, making the functional and formulative use of CA certainly preferable and advantageous with respect to NAG at equivalent concentrations and/or dosages.

Fibroblasts

From the results reported in table 14 it is possible to draw the following conclusions:

- Again, in fibroblast cultures derived from human skin, it is possible to observe a clear stimulation of protein secretion by both NAG and CA. Said stimulation reaches its peak after 24 hours remaining indistinguishable from that expressed naturally by untreated cells at 48 and 72 hours.

The appearance of the stimulatory effect is already evident at 0.5 mg/ml for NAG and at 0.66 mg/ml for CA, increasing in a dose-dependent manner (significant correlation coefficient for the concentration/effect regression in both cases, *i.e.* r=0.844 for NAG and 0.845 for CA) up to the maximum concentration tested, *i.e.* 2 mg/ml for NAG and 2.64 mg/ml for CA.

As already highlighted previously, the stimulation exerted by both test substances is very marked, reaching and exceeding, at the maximum concentrations tested, an increase of 50% with respect to the protein expression of control cultures, and thus confirming the highly positive effect exerted by both active substances on the specific functions of cells of cutaneous origin, such as fibroblasts and keratinocytes.

- As the comparison between CA and NAG concerns, again also in this case a potential difference in activity has been evaluated using the Student's "t" test, applied to the differences between the increases in percentage stimulation exerted by the two test substances, at corresponding concentrations. Said evaluation has been performed on the values measured at 24 hours since they are highly significant, thus providing statistical confirmation of the picture that has already emerged for the comparison in relation to the expression of elastin, collagen and HA by skin-derived cells, *i.e.* that CA, thanks to the unforeseen synergism between ASS and NAG, of which it is composed, is shown to be more active than NAG itself, and in a statistically significant manner, thus making functional and formulative use more advantageous.

4. **"In** vitro" evaluation of the potential cytotoxic effect of CA using the MTT test

A. On human skin-derived fibroblasts

As already explained in the descriptive section, inhibition of cellular viability has been calculated on a series of fibroblast cultures, in the presence of a set of concentrations established for CA, and by applying the formula (i) wherein "$OD_{540}$ treated cells" represents the absorbance at 540 nm of the cultures containing CA and "$OD_{540}$ untreated cells" that of the negative control (blank). Test efficiency is assessed on a culture containing a highly cytotoxic surfactant, *i.e.* sodium dodecylsulphate (SDS), at a concentration (0.05 mg/ml) at which the absorbance at 540 nm should be practically zero being the bacterial growth completely inhibited.:

$$\text{inhibition of cell viability (\%)} = \left[1 - \frac{OD540 \ cellule \ trattate}{OD540 \ cellule \ non \ trattate}\right] x \ 100 \quad \text{(i)}$$

The results obtained are reported in the following table:

**MTT test on fibroblasts in the presence of CA - Results**

| CA cone. (mg/ml) | Inhibition of cell. viability (%) Mean ± s.d. |
|---|---|
| Blank | 0.00 |
| 0.026 | 0.19 |
| 0.053 | 0.24 |
| 0.106 | 1.66 |
| 0.211 | 4.02 |
| 0.409 | 4.36 |
| 0.818 | 5.86 |
| 1.650 | 12.78 |
| 3.300 | 18.44 |

From the tabulated data, the following conclusions may be drawn:

- Test efficiency is confirmed by the fact that, following exposure of human fibroblasts to 0.5 mg/ml SDS, cell mortality of approx. 100% (96.05%) has been verified.
- As already stated, the parameter allowing the evaluation of irritant potential of a substance to be used for topical application or as an intradermal filler, is based on the determination of the $IC_{50}$, calculated from the correlation between concentration and the corresponding cell viability inhibition values, assuming the following evaluation criterion:

   ◦ $IC_{50}$ = 0.5 mg/ml: strong cytotoxic/irritant effect
   ◦ $IC_{50}$ between 0.5 and 1.5 mg/ml: moderate cytotoxic/irritant effect
   ◦ $IC_{50}$ > 1.5 mg/ml: no cytotoxic/irritant effect

In the case of CA, since at the maximum concentration tested, *i.e.* 3.30 mg/ml, the percentage inhibition of cell viability does not even reach 20% (18.44%), it is obvious that the $IC_{50}$ is much less than 1.5 mg/ml and therefore CA, according to data deduced from the "in vitro" predictive model, it may be considered devoid of any cytotoxic potential towards human skin-derived fibroblasts, and as such freely usable, both for topical use and as an intradermal filler.

B. On keratinocytes:

In the case of keratinocytes, for which the same MTT test and the same evaluation criteria, deducible from the $IC_{50}$ value, have been used, the results obtained are reported in the following table:

**MTT test on keratinocytes in the presence of CA - Results**

| CA conc. (mg/ml) | Inhibition of cell. viability (%) Mean ± s.d. |
|---|---|
| Blank | 0.00 |
| 0.026 | 0.00 |

(continued)

| CA conc. (mg/ml) | Inhibition of cell. viability (%) Mean ± s.d. |
|---|---|
| 0.053 | 0.028 |
| 0.106 | 0.056 |
| 0.211 | 0.089 |
| 0.409 | 1.166 |
| 0.818 | 6.280 |
| 1.650 | 11.330 |
| 3.300 | 17.690 |

From the tabulated data, the following conclusions may be drawn:

- Again in this case, test efficiency is confirmed by the fact that the mortality of the keratinocyte culture, exposed to 0.5 mg/ml SDS, is practically 100% (94.28%).
- With regard to the keratinocyte cultures; since, again also in this case, at the maximum concentration of CA tested (3.30 mg/ml), the % inhibition of cell vitality doesn't reach 20% and hence the $IC_{50}$ is much less than 1.5 mg/ml, CA may be considered entirely devoid of cytotoxic effects, also towards human keratinocytes.

### Conclusions

The experimental results summarised above highlight at least two fundamental considerations *i.e.,* firstly, the surprising effect associated with the combination between NAG and ASS (as both CA and as COMBI), unpredictably and unexpectedly advantageous with respect to NAG at consistent doses but without the synergic support of ASS (ASS alone has no effect), and secondly, the synergic effect between the components which reach their maximum, in the case of CA, at a ratio, expressed in terms of equivalent weight, equal to 1:1, declining progressively at values either side of this central value.

If to the above is added the complete absence of any potential cytotoxic effects of the composition according to the invention (the lack of toxicity of its components, if administered orally, being well known), it may be concluded that its potential use in association with other active ingredients, such as for example HA, preferably at doses/concentrations and according to the formulations described and claimed in the present patent application, results advantageous and safe use, both for oral administration, for example in dietary supplement formulations, and for topical application or as an intradermal filler in restoring skin tone and vigour.

### Tables

Table 1: HA expression in fibroblast cultures in the presence of ASS

| ASS conc.(*) (mg/ml) | HA(ng/well) (Mean +/- st.dev.) | HA(ng/well) (Diff.from baseline) | HA (%) (Diff.from baseline) | |
|---|---|---|---|---|
| 0.00 (baseline) | 576 +/- 52 | 0 | 0.00 | |
| 0.16 | 611 +/- 44 | 35 | +6.07 | r (corr.coeff.) (ASS conc. vs HA) -0.345 [NS(**)] |
| 0.32 | 622 +/- 91 | 46 | +7.99 | |
| 0.48 | 630 +/- 37 | 54 | +9.38 | |
| 0.64 | 427 +/- 24 | -149 | -25.87 | |

(continued)

| 0.80 | 587 +/- 56 | 11 | +1.91 | |
|------|-----------|----|-------|--|
| (*): The ASS concentration corresponds to the portion of the same component present in CA used in the same test (see Table 3 - Stage B) <br> (**) : NS = Not statistically significant | | | | |

Table 2: HA expression in fibroblast cultures in the presence of NAG

| NAG conc.(*) (mg/ml) | HA(ng/well) (Mean +/- st.dev.) | HA(ng/well) (Diff.from baseline) | HA (%) (Diff.from baseline) | |
|---|---|---|---|---|
| 0.00 (baseline) | 490 +/- 20 | 0 | 0.00 | |
| 0.50 | 544 +/- 132 | +54 | +14.02 | |
| 1.00 | 779 +/- 94 | +289 | +58.98 | r (corr.coeff.) (NAG conc. vs HA) 0.973 [SS(***)] |
| 1.50 | 863 +/- 25 | +373 | +76.12 | |
| 2.00 | 876 +/- 70 | +386 | +78.78 | |
| 2.50 | 964 +/- 192 | +474 | +96.73 | |
| (*): The NAG concentration corresponds to the portion of the same component present in the CA used in the same test (see Table 3 - Stage B) <br> (***) : SS = Statistically significant | | | | |

Table 3 - Stage A: HA expression in fibroblast cultures in the presence of NAG/ASS combinations at constant concentration and variable NAG to ASS ratios

| Conc. CA/ASS (mg/ml) | Ratio(*) NAG/ASS | HA (ng/well) from NAG/ASS (Mean +/- st.dev.) | HA(%) from NA/ASS (diff.from baseline) | HA(%) from NAG(**) (consistent doses) | Synerg.(***) NAG/ASS |
|---|---|---|---|---|---|
| 0.00 (baseline) | - | 511 +/- 18.4 | 0 | 0 | - |
| 1.98(1.75 NAG+0.23 ASS) | 1 : 0.4(1) | 843 +/- 26.9 | + 64.97 | +76.12 - +78.8 | NO |
| 1.98(1.71 NAG+0.27 ASS) | 1: 0.5(2) | 942 +/- 50.2 | +84.25 | +76.12 - +78.8 | YES(*) |
| 1.98(1.50 NAG+0.48 ASS) | 1 : 1(3) | 997 +/- 15.6 | +95.11 | +76.12 | YES(**) |
| 1.98(1.00 NAG+0.98 ASS) | 1 : 3(2) | 873 +/- 19.8 | +70.84 | +58.98 | YES(*) |

(continued)

| Conc. CA/ASS (mg/ml) | Ratio(*) NAG/ASS | HA (ng/well) from NAG/ASS (Mean +/- st.dev.) | HA(%) from NA/ASS (diff.from baseline) | HA(%) from NAG(**) (consistent doses) | Synerg.(***) NAG/ASS |
|---|---|---|---|---|---|
| 1.98(0.93 NAG+1.05 ASS) | 1 : 3.5(1) | 769 +/- 25.5 | +50.49 | ≈59 | NO |

(*): Expressed in terms of equivalent weight
(**): see Table 2
(***): NO=no synergism ; YES(+)=marked synergism ; YES(++)=maximum synergism
(1): NAG to ASS ratio outwith the range of COMBI
(2): NAG to ASS ratio within the range of COMBI
(3): NAG to ASS ratio corresponding to CA

Table 3 - Stage B: HA expression in fibroblast cultures in the presence of variable concentrations of CA

| CA conc.(*) (mg/ml) | HA(ng/well)(Mean +/- st.dev.) | HA(ng/well)(Diff.from baseline) | HA (%) (Diff.from baseline) | |
|---|---|---|---|---|
| 0.00 (baseline) | 523 +/- 31 | 0 | 0.00 | |
| 0.66(0.5 NAG+0.16 ASS) | 770 +/- 98 | +247 | +47.22 | |
| 1.32(1.0 NAG+0.32 ASS) | 884 +/- 151 | +361 | +69.02 | |
| 1.98(1.5 NAG+0.48 ASS) | 1009 +/- 86 | +486 | +92.93 | R (corr.coeff.) (CA conc. vs HA) 0.939 [SS(**)] |
| 2.64(2.0 NAG+0.64 ASS) | 1029 +/- 3 | +506 | +96.75 | |
| 3.30(2.5 NAG+0.80 ASS) | 1070 +/- 14 | +547 | +104.59 | |

(*): The CA concentration corresponds to the sum of those corresponding to NAG and $SO_4^{-2}$ of tables 1 and 2
(***): SS = Statistically significant

Table 4: HA expression in fibroblast cultures: % difference between the CA and NAG stimuli

| Ag. stimul. conc. (NAG/CA - mg/ml)(*) (1) | Expr. Of HA from CA (% incr. over baseline) (2) | Expr. of HA from NAG (% incr over baseline) (3) | CA-NAG diff. (diff.between % incr.) (2)-(3) | Student's "t" (4DoF**)=3.637 p=2.2%[(SS***) ] |
|---|---|---|---|---|
| 0.5/0.66 | +47.22 | +11.02 | +36.20 | |

(continued)

| Ag. stimul. conc. (NAG/CA - mg/ml)(*) (1) | Expr. Of HA from CA (% incr. over baseline) (2) | Expr. of HA from NAG (% incr over baseline) (3) | CA-NAG diff. (diff.between % incr.) (2)-(3) | Student's "t" (4DoF**)=3.637 p=2.2%[(SS***) ] |
|---|---|---|---|---|
| 1.0/1.32 | +69.02 | +58.98 | +10.04 | |
| 1.5/1.98 | +92.93 | +76.12 | +16.81 | |
| 2.0/2.64 | +96.75 | +78.78 | +17.97 | |
| 2.5/3.3 | +104.59 | +96.73 | +7.86 | |
| (*): The proportions between NAG and CA are consistent; e.g. 0.5 mg/ml NAG corresponding to the NAG content in 0.66 mg/ml CA. (**): DoF =Degrees of freedom (***): SS = Statistically significant | | | | |

Table 5: Collagen expression in fibroblast cultures in the presence of ASS

| ASS conc.(*) (mg/ml) | Collagen($\mu$g/ml)(Mean $^+/_-$st.dev.) | Collagen($\mu$g/ml) (Diff.from baseline) | Collagen (%) (Diff.from baseline) | |
|---|---|---|---|---|
| 0.00 (baseline) | 5.09 $^+/_-$ 0.45 | 0.00 | 0.00 | r (corr.coeff.) (ASS conc. vs collagen) -0.298 [NS(**)] |
| 0.16 | 4.66 $^+/_-$ 0.50 | -0.43 | -8.45 | |
| 0.32 | 5.29 $^+/_-$ 0.51 | +0.20 | +3.93 | |
| 0.48 | 5.18 $^+/_-$ 0.41 | +0.09 | +1.77 | |
| 0.64 | 5.55 $^+/_-$ 0.49 | +0.46 | +9.03 | |
| 0.80 | 4.92 $^+/_-$ 0.56 | -0.17 | -3.34 | |
| (*): The ASS concentration corresponds to the portion of the same component present in the CA used in the same test (see Table 7) (**): NS = Not statistically significant | | | | |

Table 6: Collagen expression in fibroblast cultures in the presence of NAG

| NAG conc.(*) (mg/ml) | Collagen($\mu$g/ml) (Mean $^+/_-$ st.dev.) | Collagen($\mu$g/ml) (Diff.from baseline) | Collagen (%) (Diff.from baseline) | r (corr.coeff.) (NAG conc. vs collagen) |
|---|---|---|---|---|
| 0.00 (baseline) | 4.26 $^+/_-$ 0.18 | 0.00 | 0.00 | |
| 0.50 | 4.33 $^+/_-$ 0.13 | +0.07 | +1.64 | |
| 1.00 | 5.55 $^+/_-$ 0.28 | +1.29 | +30.28 | |
| 1.50 | 5.80 $^+/_-$ 0.28 | +1.54 | +36.15 | |
| 2.00 | 5.66 $^+/_-$ 0.16 | +1.33 | +32.86 | |
| 2.50 | 6.29 $^+/_-$ 0.28 | +2.03 | +47.65 | |
| (*): The NAG concentration corresponds to the portion of the same component present in the CA used in the same test (see Table 7) (***): SS = Statistically significant | | | | |

Table 7: Collagen expression in fibroblast cultures in the presence of variable concentrations of CA

| CA conc.(*) (mg/ml) | Collagen($\mu$g/ml) (Mean $^+/_-$ st.dev.) | Collagen($\mu$g/ml) (Diff.from baseline) | Collagen (%) (Diff.from baseline) | r (corr.coeff.) (CA conc. vs collagen) 0.929 [SS(***)] |
|---|---|---|---|---|
| 0.00 (baseline) | 4.67 $^+/_-$ 0.22 | 0.00 | 0.00 | |
| 0.66(0.5 NAG+0.16 ASS) | 4.96 $^+/_-$ 0.19 | +0.29 | +6.22 | |
| 1.32(1.0 NAG+0.32 ASS) | 6.46 $^+/_-$ 0.23 | +1.045 | +38.48 | |
| 1.98(1.5 NAG+0.48 ASS) | 6.57 $^+/_-$ 0.24 | +1.905 | +40.84 | |
| 2.64(2.0 NAG+0.64 ASS) | 6.89 $^+/_-$ 0.25 | +2.225 | +47.70 | |
| 3.30(2.5 NAG+0.80 ASS) | 7.03 $^+/_-$ 0.27 | +2.365 | +50.70 | |
| (*): The CA concentration corresponds to the sum of those corresponding to NAG and $SO_4^{-2}$ of tables 5 and 6 (***): SS = Statistically significant | | | | |

Table 8: Elastin expression in fibroblast cultures in the presence of ASS

| ASS conc.(*) (mg/ml) | Elastin ($\mu$g/ml) (Mean $^+/_-$ st.dev.) | Elastin ($\mu$g/ml) (Diff.from baseline) | Elastin (%) (Diff.from baseline) | |
|---|---|---|---|---|
| 0.00 (baseline) | 4.72 $^+/_-$ 0.38 | 0.00 | 0.00 | |
| 0.16 | 4.61 $^+/_-$ 0.41 | -0.11 | -2.33 | r (corr.coeff.) (ASS conc. vs elastin) 0.230 [NS(**)] |
| 0.32 | 4.79 $^+/_-$ 0.44 | +0.07 | +1.98 | |
| 0.48 | 5.18 $^+/_-$ 0.36 | +0.46 | +9.75 | |
| 0.64 | 4.54 $^+/_-$ 0.47 | -0.18 | -3.81 | |
| 0.80 | 4.83 $^+/_-$ 0.30 | +0.16 | -3.39 | |
| (*): The ASS concentration corresponds to the portion of the same component present in the CA used in the same test (see Table 10) (**): NS = Not statistically significant | | | | |

Table 9: Elastin expression in fibroblast cultures in the presence of NAG

| NAG conc.(*) (mg/ml) | Elastin ($\mu$g/ml) (Mean $^+/_-$ st.dev.) | Elastin ($\mu$g/ml) (Diff.from baseline) | Elastin (%) (Diff:from baseline) | r (corr.coeff.) (MNAG conc. vs elastin) 0.859 [SS(***)] |
|---|---|---|---|---|
| 0.00 (baseline) | 5.08 $^+/_-$ 0.32 | 0.00 | 0.00 | |
| 0.50 | 16.29 $^+/_-$ 0.83 | +11.21 | +220.67 | |

(continued)

| 1.00 | 19.25 +/_ 0.99 | +14.17 | +278.94 | |
| 1.50 | 21.35+/_1.01 | +16.27 | +320.28 | |
| 2.00 | 22.62 +/_ 1.03 | +17.54 | +345.27 | |
| 2.50 | 22.30 +/_ 1.04 | +17.22 | +339.00 | |
| (*): The NAG concentration corresponds to the portion of the same component present in the CA used in the same test (see Table 10) <br> (***): SS = Statistically significant | | | | |

Table 10: Elastin expression in fibroblast cultures in the presence of variable concentrations of CA

| CA conc.(*) (mg/ml) | Elastin ($\mu$g/ml) (Mean +/_ st.dev.) | Elastin ($\mu$g/ml) (Diff.from baseline) | Elastin (%) (Diff.from baseline) | |
|---|---|---|---|---|
| 0.00 (baseline) | 5.17 +/_ 0.12 | 0.00 | 0.00 | |
| 0.66(0.5 NAG+0.16 ASS) | 14.85 +/_ 0.27 | +9.69 | +187.51 | |
| 1.32(1.0 NAG+0.32 ASS) | 20.13 +/_ 0.38 | +14.96 | +289.64 | R (corr.coeff.) (CA conc. vs elastin) 0.874 [SS(***)] |
| 1.98(1.5 NAG+0.48 ASS) | 20.19 +/_ 0.68 | +15.03 | +290.96 | |
| 2.64(2.0 NAG+0.64 ASS) | 21.36 +/_ 0.35 | +16.19 | +313.46 | |
| 3.30(2.5 NAG+0.80 ASS) | 22.72 +/_ 0.42 | +17.66 | +339.88 | |
| (*): The CA concentration corresponds to the sum of those corresponding to NAG and $SO_4^{-2}$ of tables 8 and 9 <br> (***): SS = Statistically significant | | | | |

Table 11: Collagen expression in fibroblast cultures: % difference between the CA and NAG stimuli

| Ag.stimol. conc. (NAG/CA - mg/ml)(*) (1) | Expr. of collagen from CA (% incr. over baseline) (2) | Expr. of collagen from NAG (% incr over baseline) (3) | CA-NAG diff. (diff.between % incr.) (2)-(3) | |
|---|---|---|---|---|
| 0.5/0.66 | +6.22 | +1.64 | +4.58 | Student's "t" (4DoF**)=3.34 p=2.9%[(SS***) ] |
| 1.0/1.32 | +38.48 | +30.28 | +8.20 | |
| 1.5/1.98 | +40.84 | +36.15 | +4.69 | |
| 2.0/2.64 | +47.70 | +32.86 | +14.84 | |
| 2.5/3.3 | +50.70 | +47.65 | +3.05 | |
| (*): The proportions between NAG and CA are consistent; e.g. 0.5 mg/ml NAG corresponding to the NAG content in 0.66 mg/ml CA <br> (**): DoF =Degrees of freedom <br> (***): SS = Statistically significant | | | | |

Table 12: Elastin expression in fibroblast cultures: % difference between the CA and NAG stimuli

| Ag.stimol. conc. (NAG/CA-mg/ml)(*) (1) | Expr. of elastin from CA (% incr. over baseline) (2) | Expr. of elastin from NAG (% incr over baseline) (3) | CA-NAG diff. (diff.between % incr.) (2)-(3) | Student's "t" (4DoF**)=1.78 p=14.9[(NS***) ] |
|---|---|---|---|---|
| 0.5/0.66 | +187.51 | +220.67 | -33.16 | |
| 1.0/1.32 | +289.64 | +278.94 | +10.70 | |
| 1.5/1.98 | +290.96 | +320.28 | -29.42 | |
| 2.0/2.64 | +313.46 | +345.27 | -31.81 | |
| 2.5/3.3 | +339.88 | +339.00 | +0.88 | |
| (*): The proportions between NAG and CA are consistent; e.g. 0.5 mg/ml NAG corresponding to the NAG content in 0.66 mg/ml CA<br>(**): DoF =Degrees of freedom<br>(***): NS = Not statistically significant | | | | |

Table 13: Protein expression in keratinocyte cultures in the presence of NAG and CA, and difference in % stimulation between the two substances.

| Protein Expression after 24 hours | | | | | | |
|---|---|---|---|---|---|---|
| Ag.stimol. conc. (NAG/CA - mg/ml)(*) (1) | Expr.prot. from NAG (mg/ml)-Mean +/_ st.dev. (2) | Expr.prot. from NAG % incr. over baseline (3) | Expr. prot. from CA (mg/ml)-Mean +/_ st.dev. (4) | Expr. prot. from CA % incr. over baseline (5) | CA-NAG diff. (diff.between % incr.) (5)-(3) | |
| 0.00 (baseline) | 0.245+/0.039 | 0.00 | 0.245+/0.039 | 0.00 | 0.00 | Student's "t" (3DoF**)=0.70 p=53.5% [(N***)] |
| 0.10/0.132 | 0.16+/_0.037 | -34.7 | 0.22+/_0.053 | -10.2 | +24.5 | |
| 0.50/0.66 | 0.22+/_0.039 | -10.2 | 0.255+/_0.044 | +4.1 | +14.3 | |
| 1.00/1.32 | 0.36+/_0.047 | +44.9 | 0.30+/_0.120 | +22.4 | -22.4 | |
| 2.00/2.64 | 0.38+/_0.050 | +55.1 | 0.41 +/_0.060 | +67.3 | +12.2 | |
| Protein Expression after 48 hours | | | | | | |
| 0.00 (baseline) | 0.340+/_0.11 | 0.00 | 0.340+/0.11 | 0.00 | 0.00 | Student's "t" (3Dof**)=5.74 p=1.05% [(SS****)] |
| 0.10/0.132 | 0.42+/_0.15 | +23.5 | 0.45+/_0.10 | +32.4 | +8.8 | |
| 0.50/0.66 | 0.40+/_0.12 | +17.6 | 0.47+/_0.060 | +38.2 | +20.6 | |
| 1.00/1.32 | 0.48+/_0.11 | +41.2 | 0.53+/_0.08 | +55.9 | +14.7 | |
| 2.00/2.64 | 0.47+/_0.12 | +38.2 | 0.54+/_0.08 | +58.8 | +20.6 | |
| Protein Expression after 72 hours | | | | | | |
| 0.00 (baseline) | 0.58+/_0.10 | 0.00 | 0.58+/_0.10 | 0.00 | 0.00 | |

(continued)

| Protein Expression after 72 hours | | | | | | |
|---|---|---|---|---|---|---|
| 0.10/0.132 | $0.67^{+}/_{-}0.09$ | +15.5 | $0.65^{+}/_{-}0.08$ | +12.1 | -3.4 | |
| 0.50/0.66 | $0.64^{+}/_{-}0.12$ | +10.3 | $0.63^{+}/_{-}0.09$ | +8.6 | -1.7 | |
| 1.00/1.32 | $0.70^{+}/_{-}0.08$ | +20.7 | $0.69^{+}/_{-}0.09$ | +19.0 | -1.7 | |
| 2.00/2.64 | $0.83^{+}/_{-}0.070$ | +43.1 | $0.85^{+}/_{-}0.11$ | +46.6 | +3.4 | |

(*): The proportions between NAG and CA are consistent; e.g. 0.5 mg/ml NAG corresponding to the NAG content in 0.66mg/ml CA
(**): DoF =Degrees of freedom
(***): NS = Not statistically significant
(****): SS = Statistically significant

EP 2 136 771 B1

Table 14: Protein expression in fibroblast cultures in the presence of NAG and CA, and difference in % stimulation between the two substances.

| Protein Expression after 24 hours | | | | | | |
|---|---|---|---|---|---|---|
| Ag.stimol. conc. (NAG/CA-mg/ml)(*) (1) | Expr.prot. from NAG (mg/ml)-Mean $^+/_-$ st.dev. (2) | Expr.prot. from NAG % incr. over baseline (3) | Expr. prot. from CA (mg/ml)-Mean $^+/_-$ st.dev. (4) | Expr. prot. from CA % incr. over baseline (5) | CA-NAG diff. (diff.between % incr.) (5)-(3) | |
| 0.00 (baseline) | 0.82$^+$/0.15 | 0.00 | 0.82$^+$/0.12 | 0.00 | 0.00 | Student's "t" (3Dof**)=3.7 9 p=3.2% [(SS****)] |
| 0.10/0.132 | 0.70$^+$/0.11 | -14.6 | 0.84$^+$/0.17 | +2.40 | +17.1 | |
| 0.50/0.66 | 1.08$^+$/0.29 | +31.7 | 1.14$^+$/0.21 | +39.0 | +7.3 | |
| 1.00/1.32 | 1.11$^+$/0.22 | +35.4 | 1.20$^+$/0.120 | +46.3 | +11.0 | |
| 2.00/2.64 | 1.24$^+$/0.17 | +51.2 | 1.28$^+$/0.30 | +56.1 | +4.9 | |
| Protein Expression after 48 hours | | | | | | |
| 0.00 (baseline) | 1.03$^+$/0.25 | 0.00 | 1.03$^+$/0.25 | 0.00 | 0.00 | Student's "t" (3Dof**)=1.6 8 |
| 0.10/0.132 | 0.95$^+$/0.08 | -7.8 | 1.06$^+$/0.17 | +2.9 | +10.7 | |
| 0.50/0.66 | 0.93$^+$/0.08 | -9.7 | 1.01$^+$/0.22 | -1.9 | +7.8 | p=19.3% [(NS***)] |
| 1.00/1.32 | 0.81$^+$/0.11 | -21.4 | 0.88$^+$/0.17 | -14.6 | +6.8 | |
| 2.00/2.64 | 1.10$^+$/0.20 | +6.8 | 1.06$^+$/0.20 | +2.9 | -3.9 | |
| Protein Expression after 72 hours | | | | | | |
| 0.00 (baseline) | 1.10$^+$/0.18 | 0.00 | 1.10$^+$/0.18 | 0.00 | 0.00 | Student's "t" (3Dof**)=0.2 5 p=82.1% [(NS***)] |
| 0.10/0.132 | 1.03$^+$/0.18 | -6.4 | 0.96$^+$/0.21 | -12.7 | -6.4 | |
| 0.50/0.66 | 1.14$^+$/0.16 | +3.6 | 1.13$^+$/0.20 | +2.7 | -0.9 | |
| 1.00/1.32 | 1.04$^+$/0.20 | -5.5 | 1.20$^+$/0.20 | +9.1 | +14.5 | |
| 2.00/2.64 | 1.11$^+$/0.19 | +0.91 | 1.08$^+$/0.27 | -1.8 | -2.7 | |

(*): The proportions between NAG and CA are consistent; *e.g.* 0.5 mg/ml NAG correspond to the NAG content in 0.66 mg/ml CA
(**): DoF =Degrees of freedom
(***): NS = Not statistically significant
(****): SS = Statistically significant

<u>Examples</u>

**A. Solutions and/or lipogels containing CA and non cross-linked HA to be used for topical application**

The formulations reported below, along with the description of the operating procedures used in examples 1 and 2, illustrate possible practical applications of the present invention and, since they are purely by way of example, should not be considered limiting of the invention itself in any way.

Particular reference is made to the excipients, which may be used alternatively to those explicitly mentioned in the present invention, in accordance with formulative-technological requirements, and which, in any case, may be selected from a vast range of products available on the market and well known to those skilled in the pharmaceutical sector, and hence of no inventive originality.

With regard to the dosage of active ingredients, the samples described report purely indicative quantities, any variation to which does not result in substantial modification to the methods of preparation illustrated.

More precisely, HA, expressed as sodium salt, may be advantageously used at concentrations no greater than 4% (preferably 1-3%), while CA may vary between 0.05 and 2.5% (preferably 0.1-0.5%) in accordance with the concentrations used in the "in vitro" tests, described in the experimental section and with the amount contained in the claims of the present patent application.

<u>Example 1: Monodose solution for topical application</u>

The formulation, reported in Table 1, concerns active ingredients and excipients (with description of the corresponding technological function) that may be used in the preparation of a solution for topical application, to be automatically batched into disposable containers of set volume.

The material used for the containers, for example polyethylene, must be compatible with the components of the formulation and with current standards for materials for use in the cosmetics field or for a medical device.

A preparation example of the formulation in question to be batched into monodose polyethylene containers, is hereinafter described:
- weigh out the formulation components separately according to the indications in the manufacturing instructions;
- into a dissolver, equipped with a suitable stirring system, add a quantity of double distilled water corresponding to approximately half the final weight of the solution. Start the stirring process so as to obtain a vortex and then add, in order: sodium methyl para-hydroxy-benzoate (Na Me-paraben), CA, disodium EDTA, 2-phenoxyethanol and sodium propyl para-hydroxy-benzoate (Na Pr-paraben) ensuring that each component has passed completely into solution prior to the addition of the subsequent one;
- check the pH of the resulting solution and, if necessary, adjust it with dilute sulphuric acid until a pH value equal to 7.2 $\pm$ 0.4 is obtained;
- very slowly, and with continuous vigorous stirring, add the weighed-out quantity of sodium HA and keep under vigorous stirring until complete solubilisation is reached;
- add the remaining quantity of double distilled water until the final weight is achieved;
- continue stirring for the amount of time necessary to obtain the desired viscosity (comprised of between 2500 and 4500 Pa x sec$^{-1}$ measured with shear stress equal to 5sec$^{-1}$), check the pH once more and, if necessary, adjust it with dilute sulphuric acid or sodium hydroxide;
- the resulting final solution is batched into polyethylene containers of the desired volume and shape on a dedicated automatic line.

<u>Table 1</u>: Monodose solution for topical application - composition

| Ingredient | Content (%) | Technological function |
|---|---|---|
| Sodium hyaluronate [1][2] | 1.000 | Functional ingredient |
| Condramina[3] | 1.321 | Functional ingredient |
| Na Me-paraben | 0.150 | Preservative |
| Na Pr-paraben | 0.035 | Preservative |
| 2-phenoxyethanol | 0.300 | Preservative |
| sodium EDTA | 0.100 | Coadjuvant of the preservatives |
| Dilute sulphuric acid | as req. to pH 7.2 | pH adjuster |

(continued)

| Ingredient | Content (%) | Technological function |
|---|---|---|
| Double distilled water | as req. to 100 | Solvent/carrier |

(1): Molecular weight comprised of between 0.5 and $3 \times 10^6$ Da

(2): The quantity of sodium hyaluronate weighed out may be duly adjusted according to its strength in order to comply with the stated content in the final solution.

(3): Composed of 75.7% NAG and 24.3% anhydrous sodium sulphate *i.e.* according to the weight ratio corresponding to the respective equivalent weights

Example 2: Moisturising lipogel for topical application:

The formulation, reported in Table 2, concerns active ingredients and excipients (with description of the corresponding technological function) that may be used in the preparation of a moisturising lipogel for topical application, to be batched, for example, into multidose containers. The material used for the container, for example polythene, must be compatible with the components of the formulation and with current standards for materials for use in dermo-cosmetology or for a medical device.

A preparation example of the formulation in question to be batched into multidose containers, is reported below by way of example:

- weigh out the formulation components separately according to the indications in the manufacturing instructions;

- stage 1 (preparation of the CA solution): into a suitable turbo-emulsifier, add the established quantity of purified water and CA; mix, using the blades only, for 30 minutes until a solution is obtained;

- stage 2 (aqueous phase): into the turbo-emulsifier containing the CA solution, add the glycerine, the polyethylene dimethiconol polymer, the 2-phenoxyethanol and the sodium EDTA; stir using just the blades for 30 minutes until the components are completely homogenised;

- stage 3 (oily phase): into a melting vessel, fitted with a suitable stirring system and connected by means of a vacuum transfer system to the turbo-emulsifier containing the aqueous phase, add the PEG-6 caprylic/caprycoglycerides, the Me-paraben, the Pr-paraben and stir for 20 minutes; then add the capryl carbonate and stir for further 10 minutes;

- stage 4 (emulsion formation): activate the melting vessel-turbo emulsifier connecting system, transfer stage 3 into stage 2 and use the turbo-emulsifier until a homogeneous emulsion is obtained;

- stage 5 (thickener addition): stabilise the resulting emulsion by the addition of polyacrylamide $C_{13-14}$ isoparaffin/laurylether-7 and stir for 20 minutes;

- stage 6 (silicone phase): add to the resulting stage 5, and in sequence, dimethicone/dimethicone crosspolymer and hyaluronic acid/ethyl-hexyl-palmitate, keep stirring for 20 minutes until complete homogenisation of the final gel is reached; prior to primary packaging, check the pH which must be between 5.0 and 6.0, and the viscosity, which must be between 22000 and 32000 cPs;

- stage 7 (primary packaging): the resulting gel from stage 6 may be transferred and batched on an automatic line for filling multi-purpose tubes, preferably fitted with a precision applicator. As already previously explained, the material selected for the tubes, for example polyethylene, must be compatible with the formulation components and with current standards for materials to be used in dermo-cosmetology or for a medical product.

Table 2: Moisturising lipogel for topical application - composition

| Ingredient | Content (%) | Technological function |
|---|---|---|
| Condramina[1] | 0.66 | Functional ingredient |
| HA/ethyl-hexyl-palmitate[2][3] | 1.00 | Functional ingredient |
| Glycerine | 4.00 | Humectant agent/solvent |
| 2-phenoxyethanol | 0.30 | Preservative |
| disodium EDTA | 0.10 | Chelating agent/rheologic additive |
| Dimethicone/dimethicone Crosspolymer | 20.00 | Rheologic additive |
| Polyacrylamide/$C_{13-14}$ Isoparaffin/laurylether-7 | 5.00 | Rheologic additive |
| PEG-6 caprylic/capryc glycerides | 2.00 | Emollient |
| Caprylyl carbonate | 5.00 | Emollient |

(continued)

| Ingredient | Content (%) | Technological function |
|---|---|---|
| Me-paraben | 0.15 | Preservative |
| Pr-paraben | 0.10 | Preservative |
| Polyethylene dimethiconol copolymer[4] | 3.00 | Soft-focus-filler |
| Demineralised water | as req. to 100 | Solvent/carrier |
| (1): Composed of 75.7% NAG and 24.3% anhydrous sodium sulphate *i.e.* according to the weight ratio corresponding to the respective equivalent weights<br>(2): A special composition of HA in biospheres (see Table 3) which, following application onto the surface of the skin on the face, penetrates into fine wrinkles and captures evaporation water, thus swelling with consequent natural reactivation of the skin surface. The resulting smoothing activity is also due to the external filling of the wrinkles themselves by the biospheres remaining on the surface of the skin<br>(3): The use of biospheres is an entirely optional improvement. Alternatively, they can be replaced with such quantities of HA as to obtain the same aesthetic results. HA/ethyl-hexyl-palmitate is normally commercially available<br>(4): A component consisting of special silicone elastomers, in the shape of small spherical particles, capable of uniformly reflecting incident light. The particles stick inside the furrow of the wrinkles, with a consequent surface smoothing effect and an obvious aesthetic impact. | | |

Table 3: Composition of HA/ethyl-hexyl-palmitate

| Ingredient | Content (%) | Technological function |
|---|---|---|
| LMW[*] sodium hyaluronate | 0.145 | Functional ingredient |
| HMW[**] sodium hyaluronate | 0.055 | Functional ingredient |
| Ethyl-hexyl-palmitate | 95.300 | Skin conditioner/filmogen |
| Silica dimethyl silicate | 2.500 | Thickener |
| Butylene glycol | 1.000 | Preservative |
| Caprylyl glycol | 1.000 | Preservative |
| (*): Low molecular weight<br>(**): High molecular weight | | |

**B. Visco-elastic solutions containing CA and non-crosslinked HA, to be used for skin revitalisation**

The series of operations described in example 3 must be considered purely as one of the explanatory examples of the present invention and since the procedures described have no inventive originality and do not represent any technological novelty for those skilled in the art, they must not be considered limiting of the invention itself in any way.

With regard to the concentrations and physico-chemical characteristics of the two active ingredients, the information provided for examples 1 and 2 remains valid.

Example 3: Viscoelastic solutions to be used as revitalisers for intradermal use

The formulation reported in table 4 concerns active ingredients and excipients (with descriptions of the corresponding technological functions) that may be used in the preparation of viscoelastic solutions, to be used as a medical device for intradermal use in dermo-cosmetology and aesthetic medicine.

Similarly, the packing materials used must be compatible with the formulation components and with the current regulations governing use in the production of a medical device.

A preparation example of the class of formulation in question, to be batched into monodose syringes of the desired volume, is reported below:

- weigh out the formulation components separately according to the indications in the manufacturing instructions
- Stage 1 (isotonic saline solution): in a mixer fitted with a suitable stirring system, prepare the isotonic saline solution, buffered to pH 7.2, introducing the components listed below, in the percentages described in Table 4 and stirring until a clear solution is obtained:

o Sodium chloride (NaCl)
o Sodium dihydrogen phosphate dihydrate (NaH$_2$PO$_4$•2H$_2$O)
o Disodium monohydrogen phosphate decahydrate (Na$_2$HPO$_4$•10H$_2$O)
o Water for injectables.

Check and if necessary adjust the pH to 7.2 with 1 N HCl or 1 N NaOH;

- Stage 2 (hydration): in a suitable mixer, fitted with stirring system, heating jacket and vacuum application/monitoring system, add the desired quantity of isotonic saline solution (stage 1) and the CA in such quantities as to obtain the desired concentration. Keep stirring for 10 minutes, then add the desired quantity of HA to the resulting solution and allow the mixture to stand for one day at a temperature comprised of between 2°C and 6°C, *i.e.* until complete hydration of the HA;
- stage 3 (homogenisation): having completed the hydration process, in order to avoid the incorporation of air bubbles, apply a vacuum of 0.2 bar to the mixer and leave the mass, obtained as described in the previous paragraph, to stir slowly until completely homogenised;
- prior to batching in syringes, check the osmolarity value of the resulting homogeneous mass, which must be comprised of between 260 and 360 mOsm/l;
- The final viscoelastic solution, to be administered using fine hypodermic needles (27 and 30 gauge), may be filled into monouse syringes, of the desired volume, under laminar flow in a contamination controlled environment;
- the final packaging is subsequently subjected to a sterilisation process, compatible with the formulation and the materials with which it is composed.

Table 4: Viscoelastic solution containing CA and non-crosslinked HA - composition

| Ingredient | Content (%) | Technological function |
|---|---|---|
| Hyaluronic acid[1][2] | 1.40 | Functional ingredient |
| Condramina[3] | 0.25 | Functional ingredient |
| Sodium chloride | 0.70 | Tonicity agent |
| Sodium hydrogen phosphate • 2H$_2$O | 0.0056 | Buffering agent |
| Disodium hydrogen phosphate •10 H$_2$O | 0.07 | Buffering agent |
| Water for injectables | as req. to 100 | Solvent/carrier |
| (1): Molecular weight comprised of between 0.5 and 3 x 10$^6$ Da<br>(2): The quantity of sodium hyaluronate weighed out may be duly adjusted according to its strength in order to comply with the stated content in the final solution<br>(3): Composed of 75.7% NAG and 24.3% anhydrous sodium sulphate *i.e.* according to the weight ratio corresponding to the respective equivalent weights. | | |

## C. Hydrogels containing CA and crosslinked HA to be used as intradermal filler

Examples 4 and 5 describe two procedures in which CA and crosslinked HA are incorporated into a single formulation, giving hydrogels to be used for intradermal administration.

In both cases, there is a preliminary stage, *i.e.* the crosslinking of HA, which is not covered by the claims contained in the present invention, but which is described all the same, at least in summary, in order to make the procedures reported in the examples in question comprehensible and homogeneous.

The crosslinking process described has no technological novelty for the sector, and so it is emphasised that no claimable inventive originality may be attributed to it, unlike the final hydrogels, containing crosslinked HA together with CA, never previously described and claimed due to the absolute novelty represented by the formulations including them.

The above-mentioned crosslinking process essentially consists of the formation of molecular bridges connecting the individual HA units to one another by means of the formation of covalent bonds with the bifunctional molecules of the crosslinking agent. This way, three-dimensional structures of variable consistency are formed, which, in water or in physiological liquids, have the capacity to reswell to a state of equilibrium directly in proportion to the degree of crosslinking.

On HA, there are two reaction centres, suitable for the formation of bridges by means of interaction with bifunctional agents, *i.e.:*

- the carboxyl group of the glucuronic acid, with the formation of ester bridges;
- the hydroxyl group at position 6 of the N-acetylglucosamine molecule, with the formation of ether bridges.

There is a vast range of well known crosslinking agents, widely used and easily available on the market, and from among these, we may cite by way of example: 1,4-butanediol-diglycidyl ether (BDDE), polyethylene glycol diglycidyl ether, divinylsulphone (DVS), epichlorhydrin, 1,2,3,4 di-epoxybutane, 1,2,7,8 di-epoxybutane and others.

Among the above-mentioned crosslinking agents, BDDE is preferable due to its very low toxicity and, for this reason, it is used in the formulations described and claimed in the present patent application, and consequently, in the detail of examples 4, 5.

For each crosslinking agent, there are precise practical modes of use and, in the case of BDDE, the process is achieved through the formation of ether bonds with the hydroxyl groups of NAG under the working conditions specified herein:

- ◦ BDDE/HA ratio (w/w): 1/1-1/50
- ◦ BDDE concentration in the reaction mixture: 0.5-20% (w/v)
- ◦ HA concentration in the reaction mixture: 10-20%
- ◦ Molecular weight of HA: $0.5\text{-}3\text{x}10^6$ Da
- ◦ Reaction temperature: 30-60°C
- ◦ Reaction pH: >11
- ◦ Reaction time: 2-4 hours

With regard to the dosage of active ingredients, the samples described report purely indicative quantities, and any variation to the same does not result in modification to the methods of preparation illustrated. Furthermore, such quantities are in keeping with the required conditions for the use of BDDE as a crosslinking agent, and are within the ranges already specified in examples 1, 2 and 3. For further clarity, the formulation relating to examples 4 and 5 is summarised quantitatively in table 5.

Example 4: Hydrogels of 2% HA containing 0.1% CA (CA incorporation by means of rehydration of crosslinked HA)

The process is outlined in detail by means of the steps and stages described below:

a. Preparation of the 2% crosslinked HA hydrogel

- stage 1 (dissolving the HA): in a suitable reactor, fitted with stirrer, heating jacket and vacuum control system, introduce 0.25 N NaOH and HA (as sodium salt) obtained from biofermentation (molecular weight comprised of between 0.5 and $3\text{x}10^6$ Da) in such quantities as to give a final HA concentration equal to 12% (w/v); leave the HA to hydrate for one hour at 2-6°C, then apply a vacuum of 0.2 bar, in order to avoid the incorporation of air bubbles into the mass, and start gentle stirring to as to obtain complete homogenisation;
- stage 2 (crosslinking reaction): in the reactor containing stage 1, restore atmospheric pressure, then add such a quantity of BDDE as to give a ratio of 1:16 (w/w) with respect to the HA content, and keep stirring for a further ten minutes so as to uniformly disperse the BDDE throughout the reaction mass; at this point, stop the stirring, and leave the reaction to stand for 2-4 hours at 50°C until the crosslinking process is complete; using suitable apparatus, grind the resulting gel to give particles of dimensions equal to approx. 1 cm$^3$;
- stage 3 (purification/hydration): transfer the product derived from stage 2 into a container fitted with a stirrer, prepare separately an isotonic saline solution, buffered to pH 7.2 according to the composition reported in table 6, and add such a quantity to the crosslinked HA as to give a ratio of 5:1 (w/w) with respect to the gel from stage 2; start gentle stirring and adjust to pH 7 with 1 M HCl and leave stirring for 24 hours. Repeat the operation at least three times *i.e.* until reaching reswelling equilibrium, with the attainment of an HA hydrogel with the desired concentration.

Over the course of the above-described operations, check the pH of the external isotonic solution and the osmolarity of the final hydrogel repeatedly, which must correspond to that related to the predefined HA concentration (osmolarity comprised of between 260 and 360 mOsm/l and pH comprised of between 6.8 and 7.6)

b. Preparation of the isotonic, pH 7.2 buffered solution, containing the predetermined concentration of CA (0.1%)

- in a suitable container fitted with stirrer, load the predefined quantity of saline solution buffered to pH 7.2, obtained according to the instructions given in stage 3 of step a., check the pH and, if necessary, adjust with 1 N HCl or 1 N NaOH.
- Add the predetermined quantity of CA and leave stirring until complete solubilisation. Transfer and store the solution thus obtained into a sealed container away from sources of heat.

c. Preparation of a hydrogel containing 2% of crosslinked HA and 0.1% of CA

- stage 1 (precipitation and drying of crosslinked HA): in a suitable dialysis system, fitted with a membrane adapted to the purpose (*e.g.* spectra 4 MWCO:12-14000), introduce the stated quantity of crosslinked HA hydrogel, obtained according to the description given in stage 3 of step a., transfer the dialyzer into a suitably sized container and introduce such a quantity of water as to allow exhaustive dialysis with complete elimination of the salt (replacing the dialysis medium if necessary); transfer the resulting gel into a suitable container fitted with a stirrer, add a high excess of ethanol and start vigorous stirring until the complete precipitation of the crosslinked HA; separate the solid phase by filtration and dry it, under vacuum, at a temperature no higher than 40°C; after drying and for ease of use, reduce the resulting crosslinked HA into particles of size comprised of between 20 and 50 $\mu$m by means of a suitable grinding/micronisation system;
- stage 2 (incorporation of CA by rehydration): in a suitable container fitted with a stirrer, transfer the required quantity of crosslinked HA, obtained according to the description given in stage 1, and add a suitable quantity of isotonic solution of CA buffered to pH 7.2 (see step b.), so as to give the predefined concentrations of the active ingredients, and then carry out the rehydration, while stirring very slowly, until the desired hydrogel is formed;
- stage 3 (filling into syringes): the resulting final hydrogel is suitable for batching into monodose syringes by means of a suitable filling machine, in a dedicated environment under laminar flow; the final package is subjected to a sterilisation process compatible with the formulation and the materials with which it is composed.

Example 5: Hydrogels of 2% HA containing 0.1% CA (CA incorporation by means of diffusion into the HA hydrogel)

The process is outlined in detail by means of the steps and staged described below:

- stage 1 (precipitation of the crosslinked HA hydrogel): Prepared according to the operational methods described in example 4 - step a.- stage 2;
- stage 2 (preparation of the isotonic, pH 7.2 buffered solution, containing the predetermined concentration of CA (0.1%)): prepared according to the description given in example 4 - step b.
- stage 3 (preparation of a 2% crosslinked HA and CA (0.1%) hydrogel): in an appropriate container fitted with a suitable stirring system, introduce the predefined quantity of HA hydrogel, obtained according to the operative method of stage 1, start the diffusion process by the addition of a calculated quantity of the isotonic solution of CA (see stage 2) and keep under gentle stirring for 24 hours; discard the residual isotonic solution and check that the parameters of the resulting hydrogel correspond to those established *i.e.:*

  o the osmolarity value must be comprised of between 260 and 360 mOsm/ml
  o the pH value must be comprised of between 6.8 and 7.6;
  repeat the operation for the number of times necessary and sufficient to obtain the final formulation containing crosslinked HA and CA at the established concentrations;

- stage 4 (filling into syringes): the resulting product from stage 3 is homogenised and reduced into particles of dimensions comprised of between 100 and 300 $\mu$m *i.e.* so as to be able to be batched into monodose syringes, using a suitable filling machine in a dedicated environment with laminar flow.

The final packaging is subsequently subjected to a sterilisation process, compatible with the formulation and the materials with which it is composed.

Table 5: Formulation pertaining to Examples 4 and 5

| Ingredient | Content (%) | Technological function |
|---|---|---|
| Crosslinked hyaluronic acid[(1)(2)] | 2.0 | Functional ingredient |
| Condramina[(3)] | 0.1 | Functional ingredient |
| Sodium chloride | 0.7 | Tonicity agent |
| Sodium dihydrogen phosphate • $2H_2O$ | 0.0056 | Buffering agent |
| Disodium hydrogen phosphate •$10\,H_2O$ | 0.07 | Buffering agent |
| Water for injectables | as req. to 100 | Solvent/carrier |
| (1): in sodium salt form. Molecular weight comprised of between 0.5 and $3 \times 10^6$ Da<br>(2): the quantity of sodium hyaluronate weighed out may be duly adjusted according to its strength in order to comply with the stated content in the final solution<br>(3): composed of 75.7% NAG and 24.3% anhydrous sodium sulphate *i.e.* according to the weight ratio corersponding to the respective equivalent weights. | | |

Table 6: Isotonic buffer solution at pH 7.2 - Composition

| Ingredient | Content (%) | Technological function |
|---|---|---|
| Sodium chloride | 0.7 | Isotonising agent |
| Sodium dihydrogen phosphate • $2H_2O$ | 0.0056 | Buffering agent |
| Disodium hydrogen phosphate •$10\,H_2O$ | 0.07 | Buffering agent |
| 1 N hydrochloric acid[(*)] | as required | pH adjuster |
| 1 N sodium hydroxide[(*)] | as required | pH adjuster |
| Water for injectables | as req. to 100 | Solvent/carrier |
| (*): To be used only if necessary | | |

## C. Oral forms

The formulations reported hereinafter illustrate potential practical applications of the present invention, and as such, must not be taken to be limiting of the invention itself in any way.

Particular reference is made to the excipients of the various forms under consideration, which may be used alternatively to those explicitly mentioned in the examples illustrated below, depending on the formulative-technological requirements, and which, in any case, may be selected from a vast range of products available on the market and well known to those skilled in the pharmaceutical sector, and hence of no inventive originality.

Also in the case of the oral forms, the CA dosage given in the examples is purely indicative and is in any case within the dosage range claimed in the present inventive description, *i.e.* variable between 100-1000 and preferably 250-750 mg per day, expressed in terms of glucosamine base.

The same is also true for the NAG to ASS ratio within CA, which, as previously specified, can vary freely in terms of equivalent weight between 1:0.5 and 1:3 and which, in the examples described, has been maintained constant and equal to 1:1 being the most advantageous according to the findings of the experimental section.

It should also be specified that, any variations in the dosage of CA and the content thereof in relation to NAG and ASS do not imply any substantial modifications to the preparation methods illustrated.

### Example 6: Tablets for oral use

Besides the active ingredient, the formulation reported in Table 7 comprises excipients (with a description of the corresponding technological function) that may be used in the preparation of tablets to be used for the oral administration of the subject of the present invention, alone or in association with other active ingredients.

The vehicularisation technique of the selected dose of CA, alone or in association with other active ingredients, in a tablet, requires only operations that are technologically well known and entirely normal for any operator in the art, *i.e.:*

- weighing out of the individual components of the formulation
- wet granulation, by means of an aqueous solution of polyvinylpyrrolidone K-25, NAG and microcrystalline cellulose (granulate A) in a suitable granulator
- drying of granulate A in a circulating air drying oven at a temperature no greater than 50°C to constant weight (any other type of drying may be used providing it is previously validated)
- sieving of granulate A and the other components of the formulation;
- preparation of the mixture for compression by homogenisation of granulate A with the other components of the formulation in a suitable mixer;
- compression of the resultant mixture in a suitable automated tabletting machine, with the eventual attainment of tablets of suitable shape and size.

Table 7: Example of a formulation in tablet form

| Ingredients | mg/tablet | Technological function |
|---|---|---|
| Active ingredient | | |
| Condramina[1][2] | 815.6 | Functional ingredient |
| (corresponding to glucosamine) | (500) | |
| | | |
| Excipients | | |
| Microcrystalline cellulose[2] | 61.2 | Diluent/disintegrant |
| Polyvinylpyrrolidone K-25[2] | 10.6 | Binder |
| Crosslinked sodium carboxymethyl cellulose[3] | as required | Disintegrant |
| Talc[3] | as required | Glidant/anti-caking agent |
| Mg stearate[3] | as required | Lubricant/anti-caking agent |
| Water[4] | as required | Formulative support |

(1): the quantity of CA is formulated and calculated so that the ratio between NAG and ASS, in terms of equivalent weight, is 1:1 (75.7% NAG and 24.3% ASS by weight) corresponding to a content, expressed in glucosamine base, equal to 500 mg (MW of NAG = 221.2; MW of glucosamine base = 179.2: thus, in ponderal terms, 221.2 mg of NAG corresponds to 179.17 mg of glucosamine base)
(2): used in the preparation of the CA granulate
(3): the absolute and relative quantities of said excipients depend on the size and shape of the compression die, the type of tabletting machine and the system for loading the powders into the compression chamber installed inside
(4): water is used in the preparation of granulate A and its amount depends on the components of the granulate as well as type and size of the granulator used. It is completely eliminated during drying

Example 7: Capsules for oral use

Besides the active ingredient, the formulation reported in Table 8 comprises excipients (with a description of the corresponding technological function) that may be used in the preparation of hard gelatine capsules to be used for the oral administration of the subject of the present invention, alone or optionally in association with other active ingredients.

The vehicularisation technique of the selected dose of CA, alone or in association with other active ingredients, in a hard gelatine capsule, requires only operations that are technologically well known and entirely normal for any operator skilled in the art, *i.e.:*
- weighing out of the individual components of the formulation
- preparation of the mixture to be encapsulated by dry homogenisation in a suitable mixer
- automated filling into hard gelatine capsules of suitable size and desired colour Table 8: Example of a formulation in capsule form

| Ingredients | mg/capsule | Technological function |
|---|---|---|
| Active ingredient | | |

(continued)

| Ingredients | mg/capsule | Technological function |
|---|---|---|
| Condramina[1] | 407.8 | Functional ingredient |
| (corresponding to glucosamine) | (250) | |
| | | |
| Excipients | | |
| Microcrystalline cellulose[2] | as required | Binder/Diluent |
| Talc[2] | as required | Glidant/anti-caking agent |
| Mg stearate[2] | as required | Lubricant/anti-caking agent |
| (1): see the remarks in note (1) of table 7 for calculating the dosage of CA and the mass ratios between NAG and ASS. (2): the absolute and relative quantities of the excipients depend on the size of the capsules used as administration vehicle and the dosing type/system installed in the filler used for batching the formulation into hard gelatine capsules. | | |

Example 8: Thermosealed sachets containing powders for the extemporaneous preparation of drinkable solutions/suspensions

Besides the active ingredient, the formulation reported in Table 9 comprises excipients (with a description of the corresponding technological function) that may be used in the use of the subject of the present invention, alone or optionally in association with other active ingredients, in the formulation of a powder for the preparation of extemporaneous solutions/suspensions to be taken orally.

For this purpose, the formulation in question may be suitably vehicularised in a thermosealed sachet constituted by an outer layer of paper, an aluminium interface and an inner layer f polyethylene, using preparation operations well known and useable by any technical staff operating in the specific field, *i.e.:*

- weighing out and sieving of the individual components of the formulation;
- preparation of the mixture of components by dry homogenisation in a suitable mixer;
- thermoforming of the sachets on a suitable automated line
- using the same automated line for the filling and sealing of the sachets with the desired shape and dimensions and containing the powders to be used for the preparation of solutions/suspensions to be taken orally extemporaneously.

Table 9: Example formulation in powder form for the preparation of solutions/suspensions to be taken orally extemporaneously

| Ingredients | mg/sachet | Technological function |
|---|---|---|
| Active ingredient | | |
| Condramina[1] | 815.6 | Functional ingredient |
| (corresponding to glucosamine) | (500) | |
| | | |
| Excipients | | |
| Sorbitol[2] | as required | Diluent/sweetener |
| Citric acid[2] | as required | Flavour enhancer |
| Polyethylene glycol 4000[2] | as required | Lubricant/plasticiser |
| Others[3] | as required | Sweeteners/flavourings |
| (1): see the remarks in note (1) of table 7 for calculating the dosage of CA and the mass ratios between NAG and ASS. (2): the absolute and relative quantities of said excipients depend on the type of sachet filler used and the sachet sizes. (3): flavourings and sweeteners may be added freely, depending on the organoleptic preferences. | | |

**Claims**

1. A composition useful for cosmetic and aesthetic treatment of the skin, containing N-acetylglucosamine and an alkaline metal sulphate in the equivalent weight ratio of 1:1.

2. The composition according to claim 1, wherein said alkaline metal sulphate is anhydrous sodium sulphate.

3. The composition according to claims 1 or 2, further comprising hyaluronic acid or a salt thereof.

4. The composition according to any of the claims 1 to 3, further comprising hyaluronic acid or a salt thereof in concentrations up to 4% by weight, with reference to the total weight of the composition.

5. The composition according to claim 4, comprising hyaluronic acid or a salt thereof in concentrations between 1% and 3% by weight.

6. The composition according to any of the claims 1 to 5, in a form suitable for topical or intradermal use, comprising N-acetylglucosamine and anhydrous sodium sulphate in total quantities comprised of between 0.05% and 2.5% by weight, with reference to the total weight of the composition.

7. The composition according to claim 6, comprising N-acetylglucosamine and anhydrous sodium sulphate in total quantities between 0.1% and 0.5% by weight.

8. The composition according to any of the previous claims, comprising carriers, excipients and/or preservatives for cosmetic use.

9. The composition according to any of the previous claims, in the form of a solution, lipogel or hydrogel for topical application on the skin.

10. The composition according to any of the claims 1 to 5, in the form of a unit dose for oral administration.

11. The composition according to claim 10, comprising N-acetylglucosamine and anhydrous sodium sulphate in an equivalent weight ratio of 1:1 and containing a quantity of N-acetylglucosamine comprised of between 100 and 1000 mg, expressed in terms of glucosamine base.

12. The use of a composition containing N-acetyglucosamine and an alkaline metal sulphate in equivalent weight ratio between 1:0.5 and 1:3 for the cosmetic treatment of the skin.

13. The use of claim 12, wherein N-acetylglucosamine and the alkaline metal sulphate are in the equivalent weight ratio of 1:1.

14. The use of claims 12 or 13, wherein said composition further comprises hyaluronic acid or a salt thereof.

15. An intradermal filler useful for restoring skin tone and vigour, comprising a composition containing N-acetyglucosamine and an alkaline metal sulphate in equivalent weight ratio between 1:0.5 and 1:3.

16. A dietary supplement for skin trophism, comprising a composition according to any of the claims 1 to 5.

**Patentansprüche**

1. Zusammensetzung, die für eine kosmetische und ästhetische Behandlung der Haut geeignet ist und N-Acetylglukosamin und ein Alkalimetallsulfat im Äquivalentgewichtsverhältnis von 1:1 enthält.

2. Zusammensetzung nach Anspruch 1, bei der das Alkalimetallsulfat wasserfreies Natriumsulfat ist.

3. Zusammensetzung nach Anspruch 1 oder 2, die ferner Hyaluronsäure oder ein Salz davon umfasst.

4. Zusammensetzung nach einem der Ansprüche 1 bis 3, die ferner Hyaluronsäure oder ein Salz davon in Konzentrationen bis zu 4 Gew.-% bezogen auf das Gesamtgewicht der Zusammensetzung umfasst.

5. Zusammensetzung nach Anspruch 4, die Hyaluronsäure oder ein Salz davon in Konzentrationen zwischen 1 Gew.-% und 3 Gew.-% umfasst.

6. Zusammensetzung nach einem der Ansprüche 1 bis 5 in einer Form, die für eine topische oder intradermale Verwendung geeignet ist und N-Acetylglukosamin und wasserfreies Natriumsulfat in Gesamtmengen zwischen 0,05 Gew.-% und 2,5 Gew.-% bezogen auf das Gesamtgewicht der Zusammensetzung umfasst.

7. Zusammensetzung nach Anspruch 6, die N-Acetylglukosamin und wasserfreies Natriumsulfat in Gesamtmengen zwischen 0,1 Gew.-% und 0,5 Gew.-% umfasst.

8. Zusammensetzung nach einem der vorhergehenden Ansprüche, die Träger, Hilfsstoffe und/oder Konservierungsmittel für eine kosmetische Anwendung umfasst.

9. Zusammensetzung nach einem der vorhergehenden Ansprüche in der Form einer Lösung, eines Lipogels oder eines Hydrogels für eine topische Anwendung auf der Haut.

10. Zusammensetzung nach einem der Ansprüche 1 bis 5 in der Form einer Einheitsdosis zur oralen Verabreichung.

11. Zusammensetzung nach Anspruch 10, die N-Acetylglukosamin und wasserfreies Natriumsulfat in einem Äquivalentgewichtsverhältnis von 1:1 umfasst und eine Menge an N-Acetylglukosamin zwischen 100 und 1000 mg auf der Basis von Glukosamin enthält.

12. Verwendung einer Zusammensetzung, die N-Acetylglukosamin und ein Alkalimetallsulfat im Äquivalentgewichtsverhältnis zwischen 1:0,5 und 1:3 enthält, zur kosmetischen Behandlung der Haut.

13. Verwendung nach Anspruch 12, wobei N-Acetylglukosamin und das Alkalimetallsulfat im Äquivalentgewichtsverhältnis von 1:1 vorliegen.

14. Verwendung nach Anspruch 12 oder 13, wobei die Zusammensetzung ferner Hyaluronsäure oder ein Salz davon umfasst.

15. Intradermaler Füllstoff, der zum Wiederherstellen des Hauttonus und der Hautvitalität geeignet ist und eine Zusammensetzung umfasst, die N-Acetylglukosamin und Alkalimetallsulfat im Äquivalentgewichtsverhältnis zwischen 1:0,5 und 1:3 enthält.

16. Nahrungsergänzung zur Hautversorgung, die eine Zusammensetzung nach einem der Ansprüche 1 bis 5 umfasst.


**Revendications**

1. Composition utile pour un traitement cosmétique ou esthétique de la peau, contenant de la N-acétyl-glucosamine et un sulfate de métal alcalin en un rapport en poids d'équivalents de 1/1.

2. Composition conforme à la revendication 1, dans laquelle ledit sulfate de métal alcalin est du sulfate de sodium anhydre.

3. Composition conforme à la revendication 1 ou 2, comprenant en outre de l'acide hyaluronique ou un sel de celui-ci.

4. Composition conforme à l'une des revendications 1 à 3, qui comprend en outre de l'acide hyaluronique ou un sel de celui-ci, en une concentration valant jusqu'à 4 %, en poids rapporté au poids total de la composition.

5. Composition conforme à la revendication 4, comprenant de l'acide hyaluronique ou un sel de celui-ci, en une concentration valant de 1 à 3 % en poids.

6. Composition conforme à l'une des revendications 1 à 5, sous une forme appropriée pour une utilisation topique ou intradermique, comprenant de la N-acétyl-glucosamine et du sulfate de sodium anhydre en des quantités faisant au total une proportion de 0,05 à 2,5 %, en poids rapporté au poids total de la composition.

7. Composition conforme à la revendication 6, comprenant de la N-acétyl-glucosamine et du sulfate de sodium anhydre en des quantités faisant au total une proportion de 0,1 à 0,5 % en poids.

8. Composition conforme à l'une des revendications précédentes, comprenant des véhicules, des excipients et/ou des conservateurs pour utilisation en cosmétique.

9. Composition conforme à l'une des revendications précédentes, sous forme de solution, de lipogel ou d'hydrogel, pour application en topique sur la peau.

10. Composition conforme à l'une des revendications 1 à 5, sous forme de dose unitaire pour administration par voie orale.

11. Composition conforme à la revendication 10, comprenant de la N-acétyl-glucosamine et du sulfate de sodium anhydre en un rapport en poids d'équivalents de 1/1, et contenant de la N-acétylglucosamine en une quantité, exprimée en base glucosamine, de 100 à 1000 mg.

12. Utilisation, pour un traitement cosmétique de la peau, d'une composition contenant de la N-acétyl-glucosamine et un sulfate de métal alcalin en un rapport en poids d'équivalents de 1/0,5 à 1/3.

13. Utilisation conforme à la revendication 12, dans laquelle la N-acétyl-glucosamine et le sulfate de métal alcalin se trouvent en un rapport en poids d'équivalents de 1/1.

14. Utilisation conforme à la revendication 12 ou 13, dans laquelle ladite composition comprend en outre de l'acide hyaluronique ou un sel de celui-ci.

15. Agent de remplissage intradermique utilisable pour redonner à la peau du tonus et de la vigueur, comprenant une composition contenant de la N-acétyl-glucosamine et un sulfate de métal alcalin en un rapport en poids d'équivalents de 1/0,5 à 1/3.

16. Complément diététique pour nutrition de la peau, comprenant une composition conforme à l'une des revendications 1 à 5.

**EP 2 136 771 B1**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 3697652 A
- EP 1384482 A
- EP 1075836 A